(19) Europäisches Patentamt
European Patent Office
Office européen des brevets

(11) **EP 3 543 701 A1**

(12) **EUROPEAN PATENT APPLICATION**
published in accordance with Art. 153(4) EPC

(43) Date of publication:
**25.09.2019 Bulletin 2019/39**

(21) Application number: **17864046.2**

(22) Date of filing: **26.10.2017**

(51) Int Cl.:
*G01N 33/68* (2006.01)     *C12Q 1/68* (2018.01)
*G01N 33/15* (2006.01)     *G01N 33/50* (2006.01)
*G01N 33/574* (2006.01)    *G01N 33/92* (2006.01)

(86) International application number:
**PCT/JP2017/038812**

(87) International publication number:
**WO 2018/079689 (03.05.2018 Gazette 2018/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **28.10.2016 JP 2016211239**

(71) Applicants:
• **Japanese Foundation For Cancer Research
Tokyo 135-8550 (JP)**
• **Osaka University
Suita-shi, Osaka 565-0871 (JP)**

(72) Inventors:
• **UEDA, Koji
Tokyo 135-8550 (JP)**
• **OHNISHI, Naomi
Tokyo 135-8550 (JP)**

• **NONOMURA, Norio
Suita-shi
Osaka 565-0871 (JP)**
• **UEMURA, Motohide
Suita-shi
Osaka 565-0871 (JP)**
• **FUJITA ,Kazutoshi
Suita-shi
Osaka 565-0871 (JP)**
• **TSUJIKAWA, Kazutake
Suita-shi
Osaka 565-0871 (JP)**
• **JINGUSHI, Kentarou
Suita-shi
Osaka 565-0871 (JP)**

(74) Representative: **Schön, Christoph
Dr. Schön, Neymeyr & Partner mbB
Bavariaring 26
80336 München (DE)**

(54) **BIOMARKER, METHOD FOR SEARCHING DISEASE-RELATED GENE, AND RENAL CANCER MARKER**

(57)     Provided is a method wherein an affected tissue and a normal tissue obtained from the vicinity of the affected tissue are left at rest in a culture medium, and a disease-specific biomarker is searched for in an exudate therefrom. A biomarker specific to renal cell carcinoma has been found by this method.

Figure 1

**Description**

Technical Field

The present invention relates to a method for identifying a biomarker or disease-related gene from separated/purified extracellular vesicles, and a test method. Besides, the present invention relates to a renal cancer marker obtained by using the search method.

Background Art

A biomarker refers to an in vivo substance or image data correlating with a normal process or pathological process of a body, or a pharmacological reaction against treatment, and is used as an objective index of a body condition. Biomarkers include various information such as so-called clinical laboratory values of a biochemical test, a blood test and tumor markers, and diagnostic imaging data of CT and MRI. In particular, a biomarker indicating the presence or the progression of a disease as an index of the disease is indispensable for the present medical care for finding, diagnosing and making prognostic prediction of the disease. Besides, a biomarker is used in development of a new drug for selection and evaluation of a compound to be developed.

In recent years, a large number of biomarkers have been discovered as a result of development of techniques for enabling detection of a tiny amount of protein or nucleic acid and development of genome analysis and proteome analysis. Among these, however, a very small number of markers are actually used in clinical practice. Particularly for diseases such as cancers and neurodegenerative diseases exhibiting little subjective symptoms at an early stage of the diseases, a biomarker useful as a tool for diagnosis or drug discovery is desired. Few biomarkers are, however, currently practically used.

For example, renal cell carcinoma causes few symptoms in many cases, and is found after considerable progression in most cases. Although renal cell carcinoma is recently accidentally found in more cases through ultrasonic echography or CT scan carried out for medical examination or another disease, it is still regarded as cancer difficult to find at an early stage. Patent Literatures 1 to 3 report biomarkers for renal cell carcinoma, but these biomarkers have not been put to practical use yet.

In recent years, liquid biopsy (diagnosis using a body fluid) is attracting attention in the field of clinical diagnosis using a biomarker. In the field of cancer diagnosis employing the liquid biopsy, a body liquid is used as a specimen, without collecting tumor tissue as in the conventional biopsy, for detecting a cancer cell or non-invasively or minimally invasively detecting a disease by measuring a biomarker.

As an analysis object of the liquid biopsy, a circulating tumor cell (CTC) and a DNA derived from a cancer cell (circulating tumor DNA; ctDNA) are well known. A CTC or ctDNA is, however, presumed to be detected in metastasis of a cancer cell, and hence is regarded to be suitable for prognostic prediction of cancer but unusable for early diagnosis. Therefore, as an analysis object to be used for the early diagnosis of cancers and other diseases, extracellular vesicles (hereinafter sometimes referred to as EVs) have started to attract attention.

The extracellular vesicles are vesicles released from almost all cells, and are roughly divided, depending on the size and marker molecule to be presented, into exosomes, microvesicles and apoptotic bodies. In recent years, it has been clarified that the extracellular vesicles play various roles. In particular, it has been clarified that the exosomes and the microvesicles contain nucleic acids such as mRNA and microRNA and proteins, and are involved in communication not only between close cells but also between organs (Non Patent Literatures 1 to 4).

It has been reported that the extracellular vesicles are involved, regarding cancer, in development of the cancer including angiogenesis, immunosuppression and metastasis. Besides, based on the composition of a special integrin contained in the extracellular vesicles, it has been suggested that there is a possibility of the extracellular vesicles preparing for metastasis of cancer cells (Non Patent Literature 5). In this manner, the extracellular vesicles are involved also in the development of a disease, and proteins and nucleic acids contained in the extracellular vesicles are changed depending on the state of a living body. Accordingly, a protein or a nucleic acid to be detected using the extracellular vesicles varies depending on the type or state of the disease, and the extracellular vesicles are known to work as biomarkers.

Results of studies conventionally reported are, however, based on mainly cultured cells, and there is no data of comparison of microvesicles obtained from a disease site and a normal site having the same histological background. Therefore, it has been doubted whether or not they function as a biomarker effective for disease diagnosis or new drug development.

Citation List

Patent Literature

Patent Literature 1: Japanese Patent Laid-Open No. 2016-86678
Patent Literature 2: Japanese Patent Laid-Open No. 2013-140030
Patent Literature 3: National Publication of International Patent Application No. 2015-505965

Non Patent Literature

Non Patent Literature 1: Kahlert, C., & Kalluri, R., J. Mol. Med. (Berl)., 2013, Vol. 91(4), p. 431-437.
Non Patent Literature 2: An, T. et al., J. Extracell. Vesicles., 2015, Vol. 4, 27522.
Non Patent Literature 3: Peinado, H. et al., Nat. Med., 2012, Vol. 18(6), p. 883-891
Non Patent Literature 4: Costa-Sliva, B. et al., Nat. Cell Biol., 2015, Vol. 17(6), p. 816-826.
Non Patent Literature 5: Hoshino, A. et al., Nature, 2015, Vol. 527(7578), p. 329-335.
Non Patent Literature 6: Lasser, C., et al, J. Vis. Exp., 2012, Vol. 59, e3037, doi:10.3791/3037.
Non Patent Literature 7: Geissler, K. et al., Oncoimmunology, 2015, Vol. 4, e985082.
Non Patent Literature 8: Teltsh, O., et al., Oncotarget, 2015, Vol. 32, p. 33191-33205.
Non Patent Literature 9: Guldur, M. E. et al., J. Pak. Med. Assoc., 2014, Vol. 3, p. 300-303.
Non Patent Literature 10: Bussolati, B. et al., FASEB J., 2003, Vol. 17(9), p. 1159-1161.
Non Patent Literature 11: Wiklander, O.P. et al., J. Extracell. Vesicles, 2015, Vol. 4, 26316.
Non Patent Literature 12: Tominaga, N. et al., Adv. Drug Deliv. Rev., 2015, Vol. 95, p. 50-55.
Non Patent Literature 13: Lai, C. P. et al., ACS Nano., 2014, Vol. 8(1), p. 483-494.
Non Patent Literature 14: Gruenwald, V. et al., BMC Cancer, 2010, 10:695.
Non Patent Literature 15: Vroling, L. et al., Angiogenesis, 2009, Vol. 12(1), p. 69-79.
Non Patent Literature 16: del Puerto-Nevado, L. et al., Br. J. Cancer, 2014, Vol. 110, p. 2700-2707.
Non Patent Literature 17: Shankhajit, D. et al., International Journal of Nutrition, Pharmacology, Neurological diseases, 2012, Vol. 2, p. 3-7.

Summary of Invention

Technical Problem

An object of the present invention is to provide a method for searching a novel biomarker from biological components contained in extracellular vesicles usable in liquid biopsy. Another object is to provide a novel search method for disease-related gene. Besides, a novel biomarker for renal cell carcinoma, for which an effective biomarker has not been found, and a test method are provided.

Solution to Problem

The present invention relates to a method for searching a biomarker or disease-related gene derived from diseased tissue, a test method for renal cell carcinoma, a biomarker for renal cell carcinoma, and a method for screening a therapeutic agent for renal cell carcinoma.
(1) A search method, comprising: immersing resected diseased tissue in an immersion liquid; analyzing an exuded component derived from the diseased tissue exuded from the diseased tissue in the immersion liquid; and identifying a biomarker and/or disease-related gene derived from the diseased tissue.
(2) The search method according to (1), comprising:

immersing normal tissue obtained from around the resected diseased tissue in an immersion liquid; analyzing an exuded component derived from the normal tissue exuded from the normal tissue in the immersion liquid; identifying a biomarker and/or disease-related gene derived from the normal tissue; and selecting a disease-specific biomarker and/or disease-related gene by comparatively examining the biomarker and/or disease-related gene derived from the diseased tissue and the biomarker and/or disease-related gene derived from the normal tissue.

(3) The search method according to (1) or (2), wherein the exuded component is an extracellular vesicle, a protein, a nucleic acid or a lipid.

(4) The search method according to any one of (1) to (3), wherein the exuded component is an extracellular vesicle, and the biomarker and/or disease-related gene contained in the extracellular vesicle is a protein, a nucleic acid or a lipid.

(5) The search method according to any one of (1) to (4), wherein the disease is cancer, neurodegenerative disease, multiple sclerosis, diabetes, liver disease, autism or cerebral infarction.

(6) A test method for renal cell carcinoma, comprising: separating an extracellular vesicle contained in a body fluid; detecting at least one biomarker out of biomarkers listed in Tables 1, 2 and 4 contained in the extracellular vesicle; and comparing with a prescribed value.

(7) The test method for renal cell carcinoma according to (6), wherein the biomarker is AZU1, CA9, STBD1, COMT or GYG1.

(8) The test method for renal cell carcinoma according to (6) or (7), wherein the body fluid is blood or urine.

(9) Biomarkers for renal cell carcinoma, listed in Tables 1, 2 and 4.

(10) A method for screening a therapeutic agent for renal cell carcinoma using AZU1, CA9, STBD1, COMT or GYG1 as a target, comprising: selecting a candidate compound by using AZU1, CA9, STBD1, COMT or GYG1 as an index.

Brief Description of Drawings

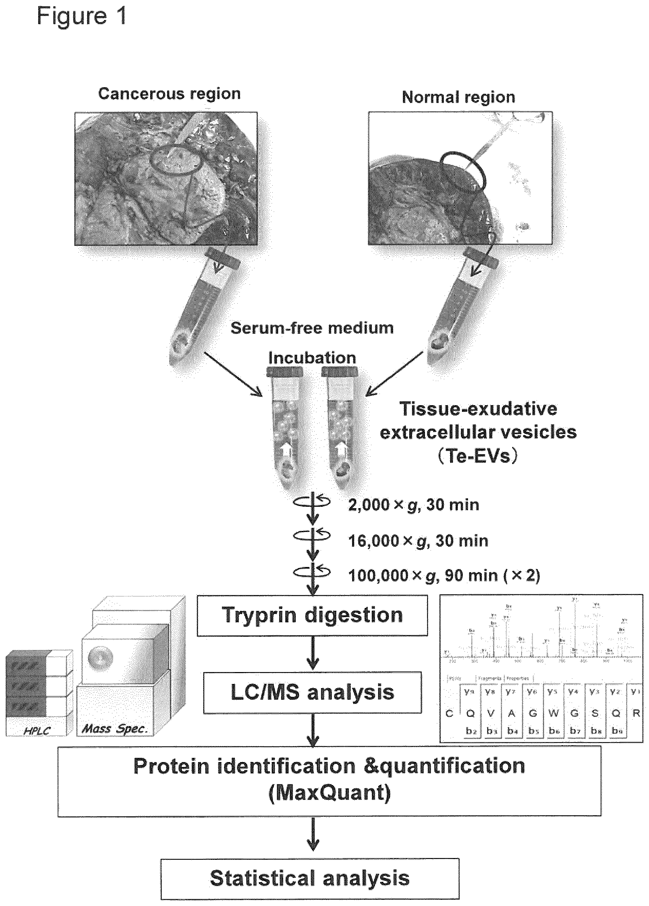

Figure 1 is a diagram illustrating the outline of a method for isolating tissue-exudative extracellular vesicles and for searching a biomarker.

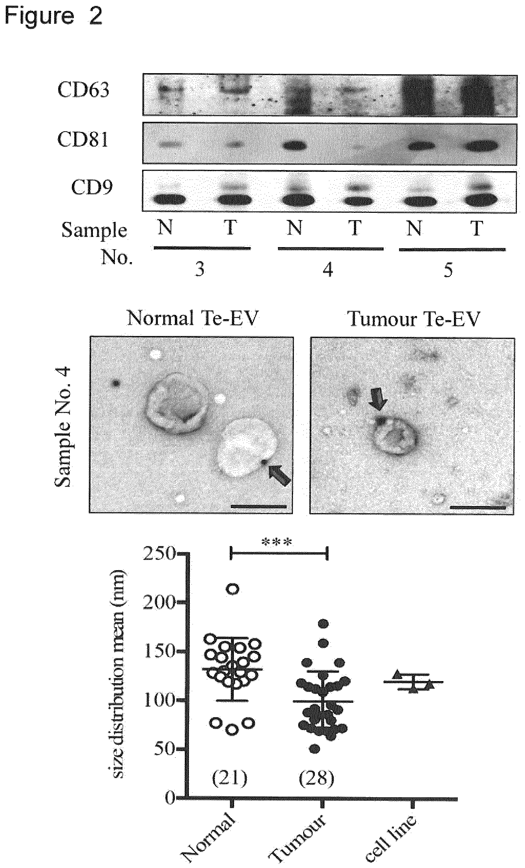

Figure 2 is a diagram illustrating marker analysis and size distribution of obtained tissue-exudative extracellular vesicles.

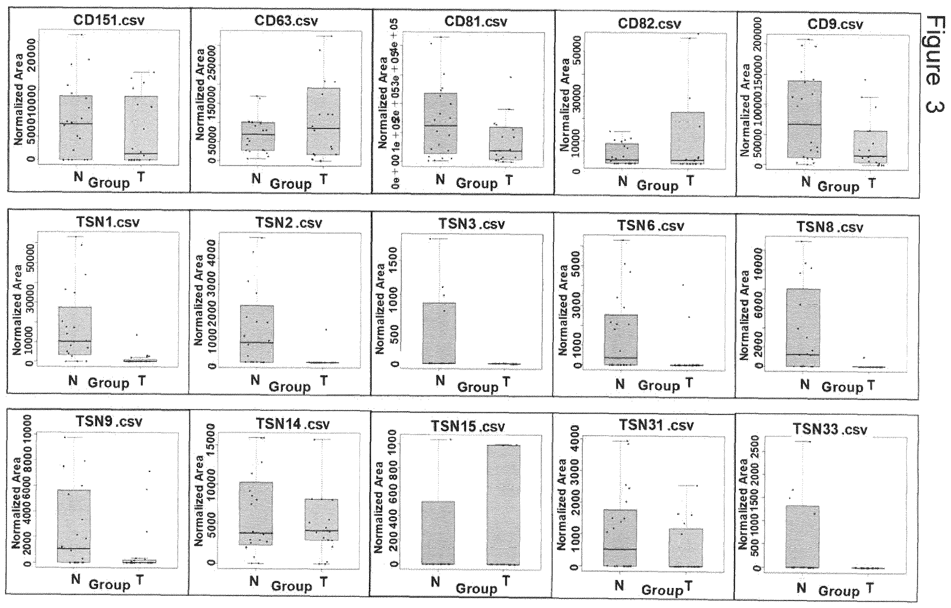

Figure 3 is a diagram illustrating the results of analysis, by mass spectrometry, of expression of tetraspanin family molecules in the tissue-exudative extracellular vesicles.

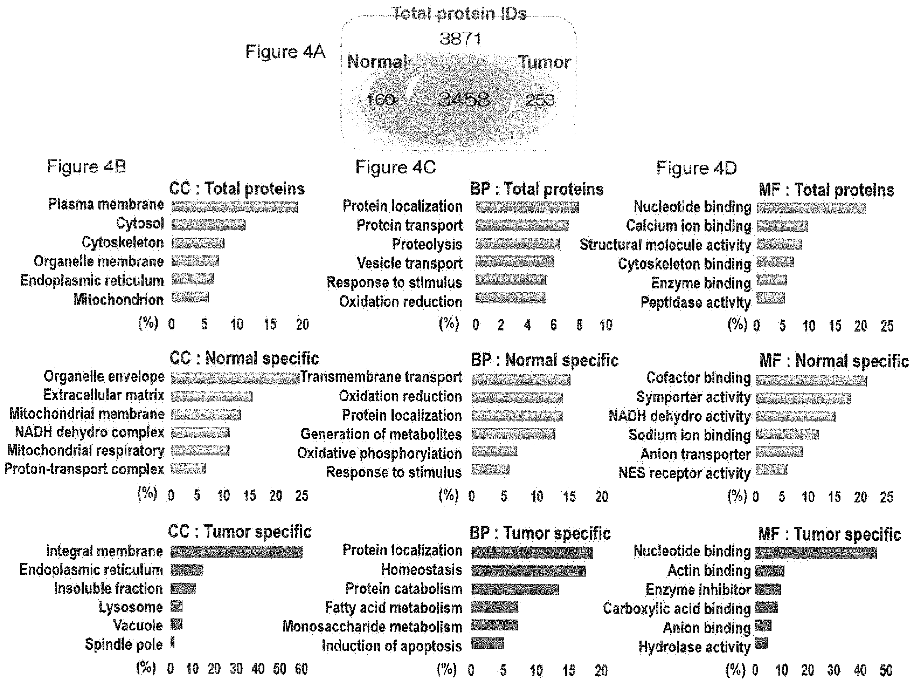

Figure 4 illustrates the analysis results of the tissue-exudative extracellular vesicles by gene ontology analysis. Figure 4A illustrates the numbers of proteins identified in tissue-exudative extracellular vesicles obtained from renal cell carcinoma tissue and normal tissue in the vicinity of the carcinoma tissue. Figure 4B, Figure 4C and Figure 4D are diagrams listing biological properties of proteins classified into three categories of cellular components, biological processes and molecular functions, respectively.

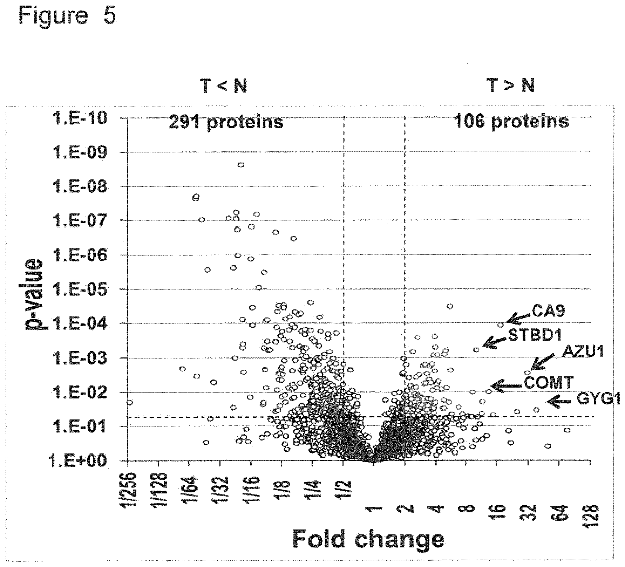

Figure 5 is a volcano plot illustrating the results of analysis, by corresponding t-test, of protein expression in extracellular vesicles derived from normal tissue and tumor tissue.

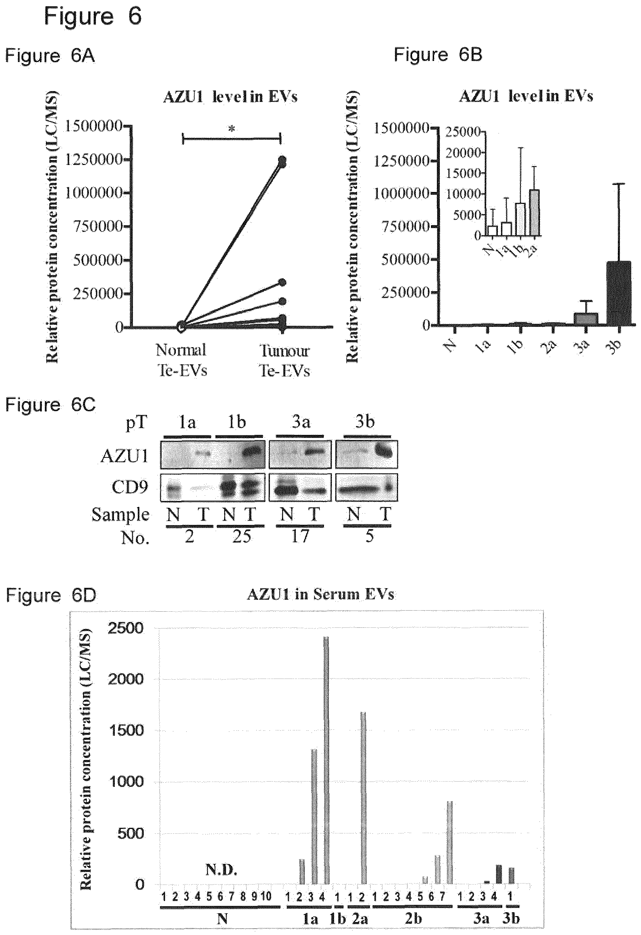

Figure 6 is a diagram illustrating expression of AZU1 in extracellular vesicles derived from normal tissue and tumor tissue. Figure 6A is a diagram illustrating the expression of AZU1 by using samples obtained from the same patient as a pair. Figure 6B is a diagram illustrating correlation between the stage of the cancer and the expression of AZU1. Figure 6C is a diagram in which the expression of AZU1 is analyzed by Western blotting. Figure 6D is a diagram illustrating an amount of AZU1 in a serum sample.

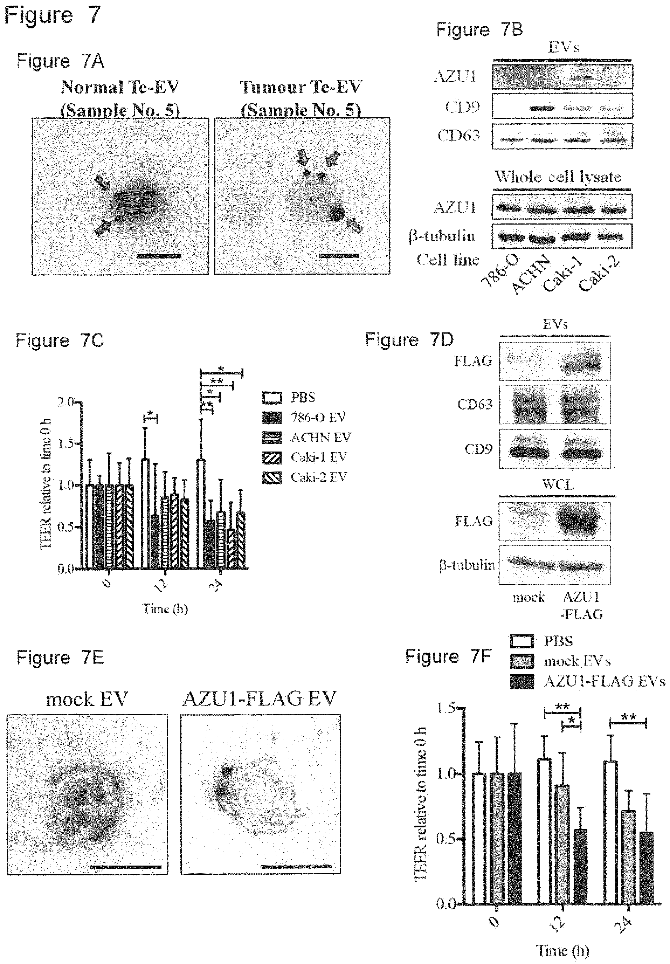

Figure 7 illustrates disintegration, depending on AZU1, of a vascular endothelial layer caused by extracellular vesicles derived from a renal cell carcinoma cell line. Figure 7A is a diagram illustrating the expression and localization of AZU1 in immunoelectron microscopic images. Figure 7B is a diagram illustrating the expression level of AZU1 and its content in secreted extracellular vesicles in the renal cell carcinoma cell line. Figure 7C is a diagram illustrating results of TEER analysis using extracellular vesicles secreted from the cell line. Figure 7D is a Western blot diagram illustrating an amount of AZU1 in the extracellular vesicles secreted from ACHN cell line in which AZU1 is forcedly expressed. Figure 7E illustrates immunoelectron microscopic images of localization of forcedly expressed AZU1. Figure 7F is a diagram illustrating results of the TEER analysis using extracellular vesicles secreted from the ACHN cell line in which AZU1 is forcedly expressed.

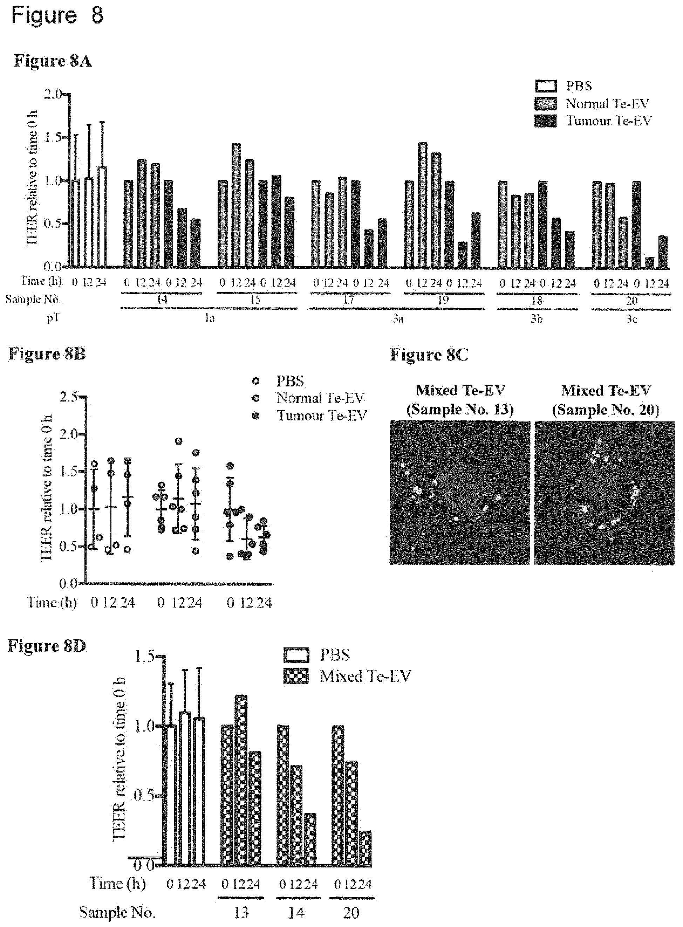

Figure 8 illustrates disintegration of a vascular endothelial layer caused by extracellular vesicles derived from a patient. Figures 8A and 8B are diagrams illustrating results of the TEER analysis using tissue-exudative extracellular vesicles derived from patient tissue. Figure 8C is a microscopic image illustrating incorporation into HUVEC cells of tissue-exudative extracellular vesicles derived from tumor tissue and normal tissue. Figure 8D is a diagram illustrating results of the TEER analysis using tissue-exudative extracellular vesicles derived from a patient.

Description of Embodiments

Now, a method for isolating extracellular vesicles from tissue and for analyzing a biomarker, and a method for searching disease-related gene will be described in detail by exemplarily describing search for a renal cell carcinoma marker, but it is noted that a search method for a biomarker and disease-related gene of the present invention is applicable to not only renal cell carcinoma but also any disease. In particular, for cancer to be treated by surgical resection of diseased tissue, a useful biomarker and disease-related gene can be searched by the method of the present invention. Besides, as the diseased tissue, not only tissue surgically obtained but also tissue obtained by autopsy can be used, and the present invention is also applicable to various diseases for which effective biomarkers have not been found yet, such as neurodegenerative diseases including amyotrophic lateral sclerosis, Parkinson's disease and Alzheimer's disease, multiple sclerosis, prostate cancer, pancreatic cancer, diabetes, liver disease, developmental disorder such as autism, and cerebral infarction.

In the case of, for example, cancer to be treated by surgical resection, a so-called non-cancerous part (normal tissue) around a cancerous part is also resected. Therefore, tissues of both a disease site and a normal site can be obtained from the same patient, and thus, samples having the same genetic background can be obtained. Accordingly, when extracellular vesicles obtained from these samples are analyzed, proteins and nucleic acids expressed irrespectively of the disease of interest in the individual can be excluded. As a result, a biomarker specific to the disease can be searched for. Besides, in the case where completely normal tissue is unavailable as in neurodegenerative disease, tissues can be obtained from a group of patients different in severity and the degree of progression to be subjected to comparative quantitative analysis, and thus, disease-related gene and a biomarker can be specified. Furthermore, a biomarker exuded from tissue and information of the biomarker obtained from a body fluid such as a serum can be used in combination.

Alternatively, in addition to the tissue obtained from a patient, tissue can be obtained from a model animal for searching a biomarker. Also for a disease such as neurodegenerative disease and diabetes for which no tissue is resected from a patient for treatment, a biomarker can be searched by using a model animal.

Besides, disease-specific biomarkers thus searched are exuded from diseased tissue, and those contained in extracellular vesicles secreted into a body fluid such as urine or blood can be selected among these to be used as a disease marker for use in liquid biopsy.

Furthermore, since a large number of biological components such as proteins are contained in extracellular vesicles, a large number of disease-specific biomarkers can be found. Among the thus obtained biomarkers, a plurality of biomarkers are selected to be measured in combination, and thus, not only diagnostic sensitivity but also specificity can be increased. When a plurality of markers are used, a disease that could not be found at an early stage can be detected.

Besides, through analysis of proteins and nucleic acids expressed specifically to a disease, a biological component that relates to the disease and can be a target of a therapeutic agent for the disease can be found. For example, when the function of a protein highly expressed in a diseased tissue is analyzed, and if it has a function essential for the onset or development of the disease, a pharmaceutical can be developed by using it as a target of a therapeutic agent.

In addition, when tissue derived from a patient who has become resistant to a molecular target drug is used, extracellular vesicles released from molecular target drug-resistant cells can be captured. A way for braking the resistance can be thus found based on an extracellular vesicle regarded as a replica of a cell.

Besides, a biomarker thus searched can be used in screening of candidate compounds in studies for developing new drugs. In high throughput screening, the screening is often carried out using a cell line. As a cell line to be used for the screening, cell lines having expression tendency similar to that of a plurality disease markers having been searched by the method of the present invention are selected in advance, and thus, candidate compounds can be accurately narrowed down. Furthermore, the obtained biomarker can be used, in addition to the cell line, for examining an effect of a candidate compound in an animal model or at a stage of clinical trial.

Now, renal cell carcinoma will be exemplarily described for describing a method for searching a novel biomarker and a test method using the same in detail. It is noted that analysis using human tissue is approved by the ethics committee of each research institution before the analysis. Besides, renal cell carcinoma tissue and normal tissue around the tumor tissue are used for the analysis after informed consent is obtained from a patient.

[Example 1] Method for Isolating Te-EVs

Figure 1 illustrates the outline of a method for isolating tissue-exudative extracellular vesicles (tissue-exudative EVs, hereinafter sometimes referred to as the Te-EVs) and analyzing a biomarker. As tissue surgically resected, diseased tissue and normal tissue are cut out and immediately immersed in an immersion liquid. In the case of renal cell carcinoma, a serum-free DMEM is used as the immersion liquid, and the immersion liquid can be appropriately selected depending on tissue from which the Te-EVs are extracted. Since extracellular vesicles are contained in a serum, a serum-free medium is preferably used as the immersion liquid, but a medium containing a serum may be used as long as extracellular vesicles are removed therefrom in advance.

A piece of tissue collected from a disease site or a normal site is allowed to stand still in the immersion liquid at 4°C for about 1 hour to cause extracellular vesicles to be secreted. The time for the standing in the immersion liquid can be adjusted in accordance with the amount of the obtained piece of tissue. When the piece of tissue is allowed to stand still in the immersion liquid for a long period of time, a larger number of extracellular vesicles can be obtained. Besides, a temperature condition for immersing the tissue can be any temperature within a range from 0°C to 37°C. Since a higher temperature increases a secretion rate and increases the amount of extracellular vesicles and the like to be obtained, the time and the temperature for the immersion may be appropriately determined. Alternatively, the immersion can be performed by, instead of still standing, gently stirring the immersion liquid by shaking, inverting or rotating. After causing extracellular vesicles to be sufficiently secreted, the resultant is centrifuged at 2,000 g for 30 minutes to remove the piece of tissue and cells through precipitation. Next, the resultant is centrifuged at 16,000 g for 30 minutes to remove cell debris through precipitation. Furthermore, a resultant supernatant is centrifuged at 100,000 g for 90 minutes to collect the Te-EVs secreted from the diseased tissue or the normal tissue through precipitation. The thus obtained Te-EVs are suspended in PBS, and washed by centrifugation at 100,000 g for 90 minutes. The thus isolated Te-EVs are extracellular vesicles derived from the diseased tissue and the normal tissue obtained from the same patient, and hence have the same genetic background, and therefore, when these are comparatively analyzed, a disease-specific biomarker can be selected.

The isolated Te-EVs are decomposed, by trypsin digestion, into peptides analyzable by mass spectrometry. The resultant peptides are identified and quantitatively determined by LC/MS (liquid chromatography/mass spectrometry) analysis. Besides, a protein specific to each of the Te-EVs is analyzed by statistical analysis.

[Example 2] Purification of Extracellular Vesicles from Renal Tissue

Te-EVs were collected to be analyzed for renal cell carcinoma patient from whom both tumor tissue and normal tissue (non-cancerous part) were obtained among 20 renal cell carcinoma patients. Histological diagnosis of renal cell carcinoma was carried out by hematoxylin-eosin staining. The disease stages of the patients classified based on AJCC TNM 6th edition were stages T1a to T3c.

The degree of purification of the thus obtained extracellular vesicles was analyzed by the Western blotting method, the immunoelectron microscopy, nanoparticle tracking analysis (Figure 2) and the mass spectrometry (Figure 3). The Western blotting method was carried out by an ordinary method using 100 ng of each Te-EVs protein obtained as above. Exosome markers of an anti-CD63 monoclonal antibody (8A12), an anti-CD81 monoclonal antibody (12C4) and an anti-CD9 monoclonal antibody (12A12) (all manufactured by Cosmo Bio Co., Ltd.) were used as primary antibodies, an HRP-labeled goat anti-mouse IgG antibody (manufactured by Santa Cruz Biotechnology, Inc.) was used as a secondary antibody, and detection was performed with ECL (ECL Prime western blotting detection reagent, manufactured by GE Healthcare).

An upper panel of Figure 2 illustrates the results of the analysis by the Western blotting method of the Te-EVs obtained from three patients. N corresponds to a non-cancerous region (normal tissue), and T corresponds to Te-EVs derived from a cancerous region. In any one of the samples, no matter whether it was obtained from a cancerous region or a non-cancerous region, expression of the exosome markers of the CD63, CD81 and CD9 was found.

The immunoelectron microscopy was performed basically in accordance with a method of Lasser et al., (Non Patent Literature 6) by using the anti-CD9 monoclonal antibody (12A12) and a 20 nm gold colloid-labeled anti-mouse antibody (manufactured by Abcam Plc.), respectively, as a primary antibody and a secondary antibody. Specifically, 1 μg of a Te-EVs sample was allowed to stand still for 1 hour on a formvar support film having carbon deposited thereon, and then was fixed with 2% paraformaldehyde to be reacted with the primary antibody. Thereafter, the resultant was reacted with the secondary antibody, and was observed with an electron microscope H-7650 (manufactured by Hitachi High-Technologies Corporation). A gold colloid bonded to the CD9 was observed as illustrated with an arrow on the Te-EVs derived from either of the normal tissue and the tumor tissue (middle panel of Figure 2).

It was found through the observation under an electron microscope that particle sizes of the Te-EVs derived from the tumor tissue largely varied. Therefore, the particle sizes of the Te-EVs derived from each tissue were measured by the nanoparticle tracking analysis. It is known that renal cell carcinoma tissue contains not only cancer cells but also non-cancerous cells (immune cells, endothelial cells and mast cells) (Non Patent Literatures 7 to 10). Therefore, it is presumed that EVs derived from normal cells are also secreted in addition to the EVs derived from cancer cells. Accordingly, in addition to the Te-EVs, extracellular vesicles secreted from cell lines established from renal cell carcinoma, that is, 786-O, ACHN and Caki-1 cell lines, were similarly isolated, purified and analyzed for a size distribution.

The 786-O, ACHN and Caki-1 cell lines were all obtained from American Type Culture Collection (ATCC), and cultured in an RPMI medium (manufactured by Wako Pure Chemical Industries Ltd.) supplemented with 10% fetal bovine serum, 100 U/mL penicillin G and 0.1 μg/mL streptomycin. For isolating EVs from the cell line, $5.0 \times 10^5$ cells were seeded in a 10 cm culture dish and cultured for 48 hours, and EVs secreted into the culture fluid were collected, by the centrifugation similarly to the Te-EVs, to be used for the analysis.

Te-EVs obtained from 21 normal tissues, Te-EVs obtained from 28 tumor tissues and EVs isolated from the above-described three renal cell carcinoma cell lines were used for analyzing the particle sizes. The nanoparticle tracking analysis was performed under the same conditions by using NanoSight LM10 (manufactured by Malvern Panalytical Ltd.) in which NTA 2.0 analysis software was installed (lower panel in Figure 2). The value of an average particle size of the EVs obtained from the renal cell carcinoma cell lines fall in a range of the particle size distribution of the Te-EVs derived from the tumor tissues, which suggested that the Te-EVs derived from the tumor tissue contain EVs secreted from cancer cells. Besides, it was clarified that the average particle size is significantly different between the Te-EVs derived from the normal tissue and those derived from the tumor tissue ($p < 0.001$). Since the particle size distribution of the Te-EVs was different between the diseased tissue and the normal tissue obtained from the renal cell carcinoma patient, there is a possibility that the particle size distribution of the Te-EVs is different between those derived from the diseased tissue and those derived from the normal tissue.

Next, Te-EVs derived from tumor tissues obtained from 20 patients and Te-EVs to be paired derived from normal tissues were used for identifying a tetraspanin molecule, which is known as an exosome marker, by the mass spectrometry, and its expression level was analyzed by the LC/MS (Figure 3).

The isolated Te-EVs were reduced with 20 mM dithiothreitol at 100°C for 10 minutes, followed by alkylation with 50 mM iodoacetamide at an ambient temperature for 45 minutes. Thereafter, the resultant was digested with 5 μl immobilized trypsin (manufactured by Thermo Fisher Scientific K.K.) by rotating/swinging at 1000 rpm at 37°C for 6 hours. The thus obtained peptide was extracted with ethyl acetate, then desalted using Oasis HLB μ-elution plate (manufactured by Waters Corporation), and subjected to the mass spectrometry. In the mass spectrometry, an LTQ-Orbitrap-Velos mass spectrometer directly connected to UltriMate 3000 RSLC nano-flow HPLC system (both manufactured by Thermo Fisher Scientific K.K.) was used. Protein was identified and quantitatively determined through analysis using MaxQuant software.

In order to examine properties of the obtained Te-EVs, the expression level of the tetraspanin molecule was compared. Fifteen tetraspanin molecules were detected, and it was revealed that the Te-EVs derived from either tissue had a high degree of purification. Besides, it was clarified that the expression of most molecules of the tetraspanin family was reduced in the Te-EVs derived from the tumor tissue as compared with the Te-EVs derived from the normal tissue.

[Example 3] Analysis of Te-EVs of Tumor Tissue and Normal Tissue in Renal Cell Carcinoma

Te-EVs derived from tumor tissue obtained from 20 patients and Te-EVs to be paired derived from normal tissue around a cancerous region were used for performing the LC/MS analysis in the same manner as described above (Figure 4). Thus, 3871 proteins in total were identified, and among these, 160 were Te-EVs derived from the normal tissue, 253 were expression specific to Te-EVs derived from the tumor tissue, and 3458 were expressed in both of these tissues (Figure 4A).

These genes were classified, in accordance with the DAVID gene ontology analysis, into categories of cellular components (CC; Figure 4B), biological processes (BP; Figure 4C) and molecular functions (MF; Figure 4D), and Figures 4B to 4D each illustrate the top 6 protein groups specific to the Te-EVs derived from the whole tissue, the normal tissue or the tumor tissue, respectively.

According to the gene ontology enrichment analysis, in the Te-EVs derived from the tumor tissue, membrane protein was characteristically present in the proteins classified as the cellular components (Figure 4B), metabolism-related protein was characteristically present in those classified as the biological processes (Figure 4C), and expression of nucleotide binding protein was characteristically present in those classified as the molecular functions (Figure 4D). That there is a difference between proteins contained in the Te-EVs derived from the tumor tissue and the those contained in the Te-EVs derived from the normal tissue means that proteins contained in the extracellular vesicles are also regulated in accordance with various cancer-related events, and indicates that these proteins are useful as cancer markers.

Among the 3871 proteins thus identified, proteins specifically expressed in the Te-EVs derived from the renal cell carcinoma tissue were analyzed. The Te-EVs derived from the tumor tissue and the normal tissue obtained from the renal tissue of the same patient were analyzed as a pair by a paired t-test. Figure 5 illustrates proteins found to be significantly different in the expression between the Te-EVs derived from the cancerous region and those derived from the non-cancerous region ($p < 0.05$, fold change $\geq 2.0$, both sides of dotted lines in Figure 5). Besides, those with large fold change are illustrated with arrows. The contents of carbonic anhydrase 9 (CA9), starch-binding domain-containing protein 1 (STBD1), azurocidin (AZU1), catechol O-methyltransferase (COMT) and glycogenin-1 (GYG1) were remarkably larger in the Te-EVs derived from the tumor tissue than in the Te-EVs derived from the normal tissue.

In the Te-EVs derived from the renal cell carcinoma tissue, as compared with the proteins contained in the Te-EVs derived from the normal tissue, the expression of 106 proteins shown in Table 1 (Tables 1-1 and 1-2) or 291 proteins shown in Table 2 (Tables 2-1 to 2-5) was found to be significantly larger or smaller.

[Table 1-1]

| AC number | Protein Description | Gene name |
|---|---|---|
| P46976 | Glycogenin-1 | GYG1 |
| P20160 | Azurocidin | AZU1 |
| P09104 | Gamma-enolase | ENO2 |
| Q16790 | Carbonic anhydrase 9 | CA9 |
| Q96HE7 | ERO1-like protein alpha | ERO1L |
| A2PYH4 | Probable ATP-dependent DNA helicase HFM1 | HFM1 |
| Q5VT79 | Annexin A8-like protein 2 | ANXA8L2 |
| P21964 | Catechol O-methyltransferase | COMT |
| O95210 | Starch-binding domain-containing protein 1 | STBD1 |
| P20701 | Integrin alpha-L | ITGAL |
| Q8N386 | Leucine-rich repeat-containing protein 25 | LRRC25 |
| O75505 | Putative double homeobox protein 2 | DUX2 |
| Q9NWQ8 | Phosphoprotein associated with glycosphingolipid-enriched microdomains 1 | PAG1 |
| P11215 | Integrin alpha-M | ITGAM |
| P32455 | Interferon-induced guanylate-binding protein 1 | GBP1 |
| P13726 | Tissue factor | F3 |
| P19971 | Thymidine phosphorylase | TYMP |
| Q8IZ83 | Aldehyde dehydrogenase family 16 member A1 | ALDH16A1 |
| P46821 | Microtubule-associated protein 1B | MAP1B |
| Q02928 | Cytochrome P450 4A11 | CYP4A11 |
| Q8IZJ1 | Netrin receptor UNC5B | UNC5B |
| Q6GTX8 | Leukocyte-associated immunoglobulin-like receptor 1 | LAIR1 |
| P04179 | Superoxide dismutase [Mn], mitochondrial | SOD2 |
| P05107 | Integrin beta-2 | ITGB2 |
| Q6NYC8 | Phostensin | PPP1R18 |
| Q14956 | Transmembrane glycoprotein NMB | GPNMB |
| Q96FQ6 | Protein S100-A16 | S100A16 |
| P32942 | Intercellular adhesion molecule 3 | ICAM3 |
| Q0JRZ9 | FCH domain only protein 2 | FCHO2 |
| P30453 | HLA class I histocompatibility antigen, A-34 alpha chain | HLA-A |
| Q8IYT3 | Coiled-coil domain-containing protein 170 | CCDC170 |
| Q06210 | Glutamine--fructose-6-phosphate aminotransferase [isomerizing] 1 | GFPT1 |
| Q96A46 | Mitoferrin-2 | SLC25A28 |
| P13807 | Glycogen [starch] synthase, muscle | GYS1 |
| Q9NZR1 | Tropomodulin-2 | TMOD2 |
| O43752 | Syntaxin-6 | STX6 |
| Q6UWP8 | Suprabasin | SBSN |
| P30273 | High affinity immunoglobulin epsilon receptor subunit gamma | FCER1G |
| P10321 | HLA class I histocompatibility antigen, Cw-7 alpha chain | HLA-C |
| Q5T681 | Uncharacterized protein C10orf62 | C10orf62 |
| P02794 | Ferritin heavy chain | FTH1 |
| P13611 | Versican core protein | VCAN |
| Q8N6N2 | Tetratricopeptide repeat protein 9B | TTC9B |
| P01892 | HLA class I histocompatibility antigen, A-2 alpha chain | HLA-A |
| P01612 | Ig kappa chain V-I region Mev | - |
| P10316 | HLA class I histocompatibility antigen, A-69 alpha chain | HLA-A |
| Q92614 | Unconventional myosin-XVIIIa | MYO18A |
| P49454 | Centromere protein F | CENPF |
| Q92572 | AP-3 complex subunit sigma-1 | AP3S1 |
| Q13643 | Four and a half LIM domains protein 3 | FHL3 |
| Q5SNT6 | WASH complex subunit FAM21B | FAM21B |
| P33908 | Mannosyl-oligosaccharide 1,2-alpha-mannosidase IA | MAN1A1 |
| P28799 | Granulins | GRN |

[Table 1-2]

| AC number | Protein Description | Gene name |
|---|---|---|
| Q01628 | Interferon-induced transmembrane protein 3 | IFITM3 |
| P20929 | Nebulin | NEB |
| Q9H2L5 | Ras association domain-containing protein 4 | RASSF4 |
| Q8TD55 | Pleckstrin homology domain-containing family O member 2 | PLEKHO2 |
| Q53T59 | HCLS1-binding protein 3 | HS1BP3 |
| P49207 | 60S ribosomal protein L34 | RPL34 |
| P31146 | Coronin-1A | CORO1A |
| P51397 | Death-associated protein 1 | DAP |
| P39060 | Collagen alpha-1(XVIII) chain | COL18A1 |
| P02786 | Transferrin receptor protein 1 | TFRC |
| Q6ZUB1 | Spermatogenesis-associated protein 31E1 | SPATA31E1 |
| P28065 | Proteasome subunit beta type-9 | PSMB9 |
| P02792 | Ferritin light chain | FTL |
| P34810 | Macrosialin | CD68 |
| P16188 | HLA class I histocompatibility antigen, A-30 alpha chain | HLA-A |
| Q9P2B2 | Prostaglandin F2 receptor negative regulator | PTGFRN |
| O00151 | PDZ and LIM domain protein 1 | PDLIM1 |
| P09972 | Fructose-bisphosphate aldolase C | ALDOC |
| Q9Y4A5 | Transformation/transcription domain-associated protein | TRRAP |
| P18084 | Integrin beta-5 | ITGB5 |
| P30508 | HLA class I histocompatibility antigen, Cw-12 alpha chain | HLA-C |
| P47914 | 60S ribosomal protein L29 | RPL29 |
| P17693 | HLA class I histocompatibility antigen, alpha chain G | HLA-G |
| P16989 | Y-box-binding protein 3 | YBX3 |
| P11169 | Solute carrier family 2, facilitated glucose transporter member 3 | SLC2A3 |
| P16949 | Stathmin | STMN1 |
| P30685 | HLA class I histocompatibility antigen, B-35 alpha chain | HLA-B |
| Q02388 | Collagen alpha-1(VII) chain | COL7A1 |
| P02747 | Complement C1q subcomponent subunit C | C1QC |
| Q9ULU4 | Protein kinase C-binding protein 1 | ZMYND8 |
| P78324 | Tyrosine-protein phosphatase non-receptor type substrate 1 | SIRPA |
| P04430 | Ig kappa chain V-I region BAN | - |
| Q7Z5R6 | Amyloid beta A4 precursor protein-binding family B member 1-interacting protein | APBB1IP |
| P01714 | Ig lambda chain V-III region SH | - |
| P06748 | Nucleophosmin | NPM1 |
| Q99571 | P2X purinoceptor 4 | P2RX4 |
| P08575 | Receptor-type tyrosine-protein phosphatase C | PTPRC |
| Q99426 | Tubulin-folding cofactor B | TBCB |
| Q9NYZ2 | Mitoferrin-1 | SLC25A37 |
| P05534 | HLA class I histocompatibility antigen, A-24 alpha chain | HLA-A |
| Q6ZRP7 | Sulfhydryl oxidase 2 | QSOX2 |
| Q16555 | Dihydropyrimidinase-related protein 2 | DPYSL2 |
| O43583 | Density-regulated protein | DENR |
| Q96MI9 | Cytosolic carboxypeptidase 4 | AGBL1 |
| P62280 | 40S ribosomal protein S11 | RPS11 |
| P27695 | DNA-(apurinic or apyrimidinic site) lyase | APEX1 |
| Q07812 | Apoptosis regulator BAX | BAX |
| O15155 | BET1 homolog | BET1 |
| P10632 | Cytochrome P450 2C8 | CYP2C8 |
| P08670 | Vimentin | VIM |
| P30622 | CAP-Gly domain-containing linker protein 1 | CLIP1 |
| P07451 | Carbonic anhydrase 3 | CA3 |
| P01703 | Ig lambda chain V-I region NEWM | - |

[Table 2-1]

| AC number | Protein Description | Gene name |
|---|---|---|
| Q8WWT9 | Solute carrier family 13 member 3 | SLC13A3 |
| P31639 | Sodium/glucose cotransporter 2 | SLC5A2 |
| O75264 | Transmembrane protein C19orf77 | C19orf77 |
| P07148 | Fatty acid-binding protein, liver | FABP1 |
| Q9UHI7 | Solute carrier family 23 member 1 | SLC23A1 |
| P16444 | Dipeptidase 1 | DPEP1 |
| P05062 | Fructose-bisphosphate aldolase B | ALDOB |
| Q9NZA1 | Chloride intracellular channel protein 5 | CLIC5 |
| P36269 | Gamma-glutamyltransferase 5 | GGT5 |
| Q03154 | Aminoacylase-1 | ACY1 |
| Q92499 | ATP-dependent RNA helicase DDX1 | DDX1 |
| Q695T7 | Sodium-dependent neutral amino acid transporter B(0)AT1 | SLC6A19 |
| Q13113 | PDZK1-interacting protein 1 | PDZK1IP1 |
| O00592 | Podocalyxin | PODXL |
| P09467 | Fructose-1,6-bisphosphatase 1 | FBP1 |
| P21695 | Glycerol-3-phosphate dehydrogenase [NAD(+)], cytoplasmic | GPD1 |
| Q5T2W1 | Na(+)/H(+) exchange regulatory cofactor NHE-RF3 | PDZK1 |
| Q15599 | Na(+)/H(+) exchange regulatory cofactor NHE-RF2 | SLC9A3R2 |
| Q9BYF1 | Angiotensin-converting enzyme 2 | ACE2 |
| Q8WW52 | Protein FAM151A | FAM151A |
| P51580 | Thiopurine S-methyltransferase | TPMT |
| O96013 | Serine/threonine-protein kinase PAK 4 | PAK4 |
| Q93088 | Betaine--homocysteine S-methyltransferase 1 | BHMT |
| P07911 | Uromodulin | UMOD |
| P29972 | Aquaporin-1 | AQP1 |
| Q07837 | Neutral and basic amino acid transport protein rBAT | SLC3A1 |
| P08473 | Neprilysin | MME |
| P15144 | Aminopeptidase N | ANPEP |
| P35558 | Phosphoenolpyruvate carboxykinase, cytosolic [GTP] | PCK1 |
| O43175 | D-3-phosphoglycerate dehydrogenase | PHGDH |
| P08729 | Keratin, type II cytoskeletal 7 | KRT7 |
| O14745 | Na(+)/H(+) exchange regulatory cofactor NHE-RF1 | SLC9A3R1 |
| P09758 | Tumor-associated calcium signal transducer 2 | TACSTD2 |
| P00742 | Coagulation factor X | F10 |
| Q9UGT4 | Sushi domain-containing protein 2 | SUSD2 |
| Q1EHB4 | Sodium-coupled monocarboxylate transporter 2 | SLC5A12 |
| O75348 | V-type proton ATPase subunit G 1 | ATP6V1G1 |
| Q16864 | V-type proton ATPase subunit F | ATP6V1F |
| Q14894 | Thiomorpholine-carboxylate dehydrogenase | CRYM |
| Q9Y2J2 | Band 4.1-like protein 3 | EPB41L3 |
| Q4V9L6 | Transmembrane protein 119 | TMEM119 |
| P11465 | Pregnancy-specific beta-1-glycoprotein 2 | PSG2 |
| Q9H0W9 | Ester hydrolase C11orf54 | C11orf54 |
| P12821 | Angiotensin-converting enzyme | ACE |
| P13640 | Metallothionein-1G | MT1G |
| P21266 | Glutathione S-transferase Mu 3 | GSTM3 |
| P52758 | Ribonuclease UK114 | HRSP12 |
| P16083 | Ribosyldihydronicotinamide dehydrogenase [quinone] | NQO2 |
| Q6ZQN7 | Solute carrier organic anion transporter family member 4C1 | SLCO4C1 |
| P36543 | V-type proton ATPase subunit E 1 | ATP6V1E1 |
| O75954 | Tetraspanin-9 | TSPAN9 |
| O43451 | Maltase-glucoamylase, intestinal | MGAM |
| P11137 | Microtubule-associated protein 2 | MAP2 |
| P15941 | Mucin-1 | MUC1 |
| Q9NQ84 | G-protein coupled receptor family C group 5 member C | GPRC5C |
| P07305 | Histone H1.0 | H1F0 |
| P53990 | IST1 homolog | IST1 |
| P05937 | Calbindin | CALB1 |

[Table 2-2]

| AC number | Protein Description | Gene name |
|---|---|---|
| P50135 | Histamine N-methyltransferase | HNMT |
| O75131 | Copine-3 | CPNE3 |
| O60262 | Guanine nucleotide-binding protein G(I)/G(S)/G(O) subunit gamma-7 | GNG7 |
| Q9Y2S2 | Lambda-crystallin homolog | CRYL1 |
| Q16822 | Phosphoenolpyruvate carboxykinase [GTP], mitochondrial | PCK2 |
| P62330 | ADP-ribosylation factor 6 | ARF6 |
| Q16625 | Occludin | OCLN |
| P22748 | Carbonic anhydrase 4 | CA4 |
| P18859 | ATP synthase-coupling factor 6, mitochondrial | ATP5J |
| O43895 | Xaa-Pro aminopeptidase 2 | XPNPEP2 |
| P27487 | Dipeptidyl peptidase 4 | DPP4 |
| Q9UI12 | V-type proton ATPase subunit H | ATP6V1H |
| P29034 | Protein S100-A2 | S100A2 |
| Q01740 | Dimethylaniline monooxygenase [N-oxide-forming] 1 | FMO1 |
| P07195 | L-lactate dehydrogenase B chain | LDHB |
| P38606 | V-type proton ATPase catalytic subunit A | ATP6V1A |
| Q8N357 | Solute carrier family 35 member F6 | SLC35F6 |
| Q14019 | Coactosin-like protein | COTL1 |
| Q9BXI6 | TBC1 domain family member 10A | TBC1D10A |
| P09669 | Cytochrome c oxidase subunit 6C | COX6C |
| Q9BUT1 | 3-hydroxybutyrate dehydrogenase type 2 | BDH2 |
| P21281 | V-type proton ATPase subunit B, brain isoform | ATP6V1B2 |
| O76094 | Signal recognition particle subunit SRP72 | SRP72 |
| Q00796 | Sorbitol dehydrogenase | SORD |
| Q2LD37 | Uncharacterized protein KIAA1109 | KIAA1109 |
| Q96C23 | Aldose 1-epimerase | GALM |
| Q9Y696 | Chloride intracellular channel protein 4 | CLIC4 |
| Q12929 | Epidermal growth factor receptor kinase substrate 8 | EPS8 |
| P30086 | Phosphatidylethanolamine-binding protein 1 | PEBP1 |
| O60749 | Sorting nexin-2 | SNX2 |
| Q9BYE9 | Cadherin-related family member 2 | CDHR2 |
| B2RUZ4 | Small integral membrane protein 1 | SMIM1 |
| P05413 | Fatty acid-binding protein, heart | FABP3 |
| P17813 | Endoglin | ENG |
| P00966 | Argininosuccinate synthase | ASS1 |
| Q96FL8 | Multidrug and toxin extrusion protein 1 | SLC47A1 |
| Q92820 | Gamma-glutamyl hydrolase | GGH |
| O43181 | NADH dehydrogenase [ubiquinone] iron-sulfur protein 4, mitochondrial | NDUFS4 |
| Q13228 | Selenium-binding protein 1 | SELENBP1 |
| Q9HBJ8 | Collectrin | TMEM27 |
| P13073 | Cytochrome c oxidase subunit 4 isoform 1, mitochondrial | COX4I1 |
| P09210 | Glutathione S-transferase A2 | GSTA2 |
| P35241 | Radixin | RDX |
| P22732 | Solute carrier family 2, facilitated glucose transporter member 5 | SLC2A5 |
| Q9Y5X3 | Sorting nexin-5 | SNX5 |
| P98082 | Disabled homolog 2 | DAB2 |
| Q07075 | Glutamyl aminopeptidase | ENPEP |
| P21291 | Cysteine and glycine-rich protein 1 | CSRP1 |
| P29992 | Guanine nucleotide-binding protein subunit alpha-11 | GNA11 |
| P82980 | Retinol-binding protein 5 | RBP5 |
| Q9UBI6 | Guanine nucleotide-binding protein G(I)/G(S)/G(O) subunit gamma-12 | GNG12 |
| Q13277 | Syntaxin-3 | STX3 |
| O43490 | Prominin-1 | PROM1 |
| O95747 | Serine/threonine-protein kinase OSR1 | OXSR1 |
| Q9HD42 | Charged multivesicular body protein 1a | CHMP1A |
| Q8NGM8 | Olfactory receptor 6M1 | OR6M1 |
| P05026 | Sodium/potassium-transporting ATPase subunit beta-1 | ATP1B1 |
| Q9NVS9 | Pyridoxine-5-phosphate oxidase | PNPO |
| P21912 | Succinate dehydrogenase [ubiquinone] iron-sulfur subunit, mitochondrial | SDHB |

[Table 2-3]

| AC number | Protein Description | Gene name |
|---|---|---|
| P16930 | Fumarylacetoacetase | FAH |
| P08195 | 4F2 cell-surface antigen heavy chain | SLC3A2 |
| P14550 | Alcohol dehydrogenase [NADP(+)] | AKR1A1 |
| Q9H0E2 | Toll-interacting protein | TOLLIP |
| P56385 | ATP synthase subunit e, mitochondrial | ATP5I |
| Q9Y5K8 | V-type proton ATPase subunit D | ATP6V1D |
| Q93099 | Homogentisate 1,2-dioxygenase | HGD |
| Q9Y6R1 | Electrogenic sodium bicarbonate cotransporter 1 | SLC4A4 |
| Q16853 | Membrane primary amine oxidase | AOC3 |
| P54710 | Sodium/potassium-transporting ATPase subunit gamma | FXYD2 |
| P34896 | Serine hydroxymethyltransferase, cytosolic | SHMT1 |
| Q9NVD7 | Alpha-parvin | PARVA |
| Q7Z3B1 | Neuronal growth regulator 1 | NEGR1 |
| P12277 | Creatine kinase B-type | CKB |
| O95292 | Vesicle-associated membrane protein-associated protein B/C | VAPB |
| P63000 | Ras-related C3 botulinum toxin substrate 1 | RAC1 |
| O15244 | Solute carrier family 22 member 2 | SLC22A2 |
| Q92736 | Ryanodine receptor 2 | RYR2 |
| Q13427 | Peptidyl-prolyl cis-trans isomerase G | PPIG |
| O96019 | Actin-like protein 6A | ACTL6A |
| P50148 | Guanine nucleotide-binding protein G(q) subunit alpha | GNAQ |
| P35611 | Alpha-adducin | ADD1 |
| Q99653 | Calcineurin B homologous protein 1 | CHP1 |
| P00167 | Cytochrome b5 | CYB5A |
| Q8NFU3 | Thiosulfate sulfurtransferase/rhodanese-like domain-containing protein 1 | TSTD1 |
| P19440 | Gamma-glutamyltranspeptidase 1 | GGT1 |
| Q96IX5 | Up-regulated during skeletal muscle growth protein 5 | USMG5 |
| P09455 | Retinol-binding protein 1 | RBP1 |
| P15311 | Ezrin | EZR |
| O94760 | N(G),N(G)-dimethylarginine dimethylaminohydrolase 1 | DDAH1 |
| O00499 | Myc box-dependent-interacting protein 1 | BIN1 |
| P48059 | LIM and senescent cell antigen-like-containing domain protein 1 | LIMS1 |
| Q16775 | Hydroxyacylglutathione hydrolase, mitochondrial | HAGH |
| P05023 | Sodium/potassium-transporting ATPase subunit alpha-1 | ATP1A1 |
| Q8WU39 | Marginal zone B- and B1-cell-specific protein | MZB1 |
| P80723 | Brain acid soluble protein 1 | BASP1 |
| P54920 | Alpha-soluble NSF attachment protein | NAPA |
| O75309 | Cadherin-16 | CDH16 |

(continued)

| AC number | Protein Description | Gene name |
|---|---|---|
| P62873 | Guanine nucleotide-binding protein G(I)/G(S)/G(T) subunit beta-1 | GNB1 |
| P98164 | Low-density lipoprotein receptor-related protein 2 | LRP2 |
| Q9Y2Q5 | Ragulator complex protein LAMTOR2 | LAMTOR2 |
| P20073 | Annexin A7 | ANXA7 |
| Q96IU4 | Alpha/beta hydrolase domain-containing protein 14B | ABHD14B |
| Q9BZV1 | UBX domain-containing protein 6 | UBXN6 |
| O75936 | Gamma-butyrobetaine dioxygenase | BBOX1 |
| P50053 | Ketohexokinase | KHK |
| P00918 | Carbonic anhydrase 2 | CA2 |
| P11279 | Lysosome-associated membrane glycoprotein 1 | LAMP1 |
| O75874 | Isocitrate dehydrogenase [NADP] cytoplasmic | IDH1 |
| P00325 | Alcohol dehydrogenase 1 B | ADH1B |
| Q14699 | Raftlin | RFTN1 |
| Q8N201 | Integrator complex subunit 1 | INTS1 |
| Q9NZZ3 | Charged multivesicular body protein 5 | CHMP5 |
| Q8WUM4 | Programmed cell death 6-interacting protein | PDCD6IP |
| P09211 | Glutathione S-transferase P | GSTP1 |
| Q10567 | AP-1 complex subunit beta-1 | AP1B1 |
| Q96KP4 | Cytosolic non-specific dipeptidase | CNDP2 |
| Q9H1C7 | Cysteine-rich and transmembrane domain-containing protein 1 | CYSTM1 |
| P60953 | Cell division control protein 42 homolog | CDC42 |

[Table 2-4]

| AC number | Protein Description | Gene name |
|---|---|---|
| P24539 | ATP synthase subunit b, mitochondrial | ATP5F1 |
| P62070 | Ras-related protein R-Ras2 | RRAS2 |
| Q9UHE5 | Probable N-acetyltransferase 8 | NAT8 |
| Q8NFJ5 | Retinoic acid-induced protein 3 | GPRC5A |
| Q13596 | Sorting nexin-1 | SNX1 |
| Q8N3Y1 | F-box/WD repeat-containing protein 8 | FBXW8 |
| Q9NZ45 | CDGSH iron-sulfur domain-containing protein 1 | CISD1 |
| Q9H4A4 | Aminopeptidase B | RNPEP |
| Q96DG6 | Carboxymethylenebutenolidase homolog | CMBL |
| Q9ULE6 | Paladin | PALD1 |
| Q6UX53 | Methyltransferase-like protein 7B | METTL7B |
| P17174 | Aspartate aminotransferase, cytoplasmic | GOT1 |
| P55795 | Heterogeneous nuclear ribonucleoprotein H2 | HNRNPH2 |

(continued)

| AC number | Protein Description | Gene name |
|---|---|---|
| Q9NQR4 | Omega-amidase NIT2 | NIT2 |
| P51149 | Ras-related protein Rab-7a | RAB7A |
| P11908 | Ribose-phosphate pyrophosphokinase 2 | PRPS2 |
| P10599 | Thioredoxin | TXN |
| P05783 | Keratin, type I cytoskeletal 18 | KRT18 |
| O94903 | Proline synthase co-transcribed bacterial homolog protein | PROSC |
| Q9UJ68 | Mitochondrial peptide methionine sulfoxide reductase | MSRA |
| P15374 | Ubiquitin carboxyl-terminal hydrolase isozyme L3 | UCHL3 |
| P32119 | Peroxiredoxin-2 | PRDX2 |
| Q9NVA2 | Septin-11 | SEPT11 |
| Q6QHC5 | Sphingolipid delta(4)-desaturase/C4-hydroxylase DES2 | DEGS2 |
| P11233 | Ras-related protein Ral-A | RALA |
| Q96CX2 | BTB/POZ domain-containing protein KCTD12 | KCTD12 |
| O95154 | Aflatoxin B1 aldehyde reductase member 3 | AKR7A3 |
| Q13183 | Solute carrier family 13 member 2 | SLC13A2 |
| P84077 | ADP-ribosylation factor 1 | ARF1 |
| P21810 | Biglycan | BGN |
| P21796 | Voltage-dependent anion-selective channel protein 1 | VDAC1 |
| Q16270 | Insulin-like growth factor-binding protein 7 | IGFBP7 |
| P62834 | Ras-related protein Rap-1A | RAP1A |
| P13473 | Lysosome-associated membrane glycoprotein 2 | LAMP2 |
| Q00839 | Heterogeneous nuclear ribonucleoprotein U | HNRNPU |
| Q13418 | Integrin-linked protein kinase | ILK |
| P51148 | Ras-related protein Rab-5C | RAB5C |
| P50895 | Basal cell adhesion molecule | BCAM |
| P62820 | Ras-related protein Rab-1A | RAB1A |
| Q96F10 | Diamine acetyltransferase 2 | SAT2 |
| P21283 | V-type proton ATPase subunit C 1 | ATP6V1C1 |
| Q9HCU5 | Prolactin regulatory element-binding protein | PREB |
| P68104 | Elongation factor 1-alpha 1 | EEF1A1 |
| Q9NQV5 | PR domain-containing protein 11 | PRDM11 |
| Q9UEU0 | Vesicle transport through interaction with t-SNAREs homolog 1B | VTI1B |
| Q9H2A2 | Aldehyde dehydrogenase family 8 member A1 | ALDH8A1 |
| Q01650 | Large neutral amino acids transporter small subunit 1 | SLC7A5 |
| P11142 | Heat shock cognate 71 kDa protein | HSPA8 |
| Q9NRA2 | Sialin | SLC17A5 |
| O75165 | DnaJ homolog subfamily C member 13 | DNAJC13 |
| P61224 | Ras-related protein Rap-1b | RAP1B |

(continued)

| AC number | Protein Description | Gene name |
|---|---|---|
| P17927 | Complement receptor type 1 | CR1 |
| Q96A57 | Transmembrane protein 230 | TMEM230 |
| O94886 | Transmembrane protein 63A | TMEM63A |
| P60981 | Destrin | DSTN |
| P30153 | Serine/threonine-protein phosphatase 2A65 kDa regulatory subunit A alpha isoform | PPP2R1A |
| Q06830 | Peroxiredoxin-1 | PRDX1 |
| Q96BW5 | Phosphotriesterase-related protein | PTER |
| Q6P4A3 | Phospholipase B-like 1 | PLBD1 |

[Table 2-5]

| AC number | Protein Description | Gene name |
|---|---|---|
| P04216 | Thy-1 membrane glycoprotein | THY1 |
| P56199 | Integrin alpha-1 | ITGA1 |
| O95865 | N(G),N(G)-dimethylarginine dimethylaminohydrolase 2 | DDAH2 |
| Q6IAA3 | Ragulator complex protein LAMTOR1 | LAMTOR1 |
| P40925 | Malate dehydrogenase, cytoplasmic | MDH1 |
| O00560 | Syntenin-1 | SDCBP |
| P54707 | Potassium-transporting ATPase alpha chain 2 | ATP12A |
| Q02952 | A-kinase anchor protein 12 | AKAP12 |
| P08183 | Multidrug resistance protein 1 | ABCB1 |
| Q9Y4F1 | FERM, RhoGEF and pleckstrin domain-containing protein 1 | FARP1 |
| P02549 | Spectrin alpha chain, erythrocytic 1 | SPTA1 |
| Q5TZA2 | Rootletin | CROCC |
| P01116 | GTPase KRas | KRAS |
| Q15907 | Ras-related protein Rab-11B | RAB11B |
| Q9H2P9 | Diphthine synthase | DPH5 |
| O75223 | Gamma-glutamylcyclotransferase | GGCT |
| Q9H223 | EH domain-containing protein 4 | EHD4 |
| P30041 | Peroxiredoxin-6 | PRDX6 |
| P30046 | D-dopachrome decarboxylase | DDT |
| P22352 | Glutathione peroxidase 3 | GPX3 |
| Q13200 | 26S proteasome non-ATPase regu latory subunit 2 | PSMD2 |
| P62879 | Guanine nucleotide-binding protein G(I)/G(S)/G(T) subunit beta-2 | GNB2 |
| P00441 | Superoxide dismutase [Cu-Zn] | SOD1 |
| P98172 | Ephrin-B1 | EFNB1 |
| Q13526 | Peptidyl-prolyl cis-trans isomerase NIMA-interacting 1 | PIN1 |
| P15090 | Fatty acid-binding protein, adipocyte | FABP4 |

(continued)

| AC number | Protein Description | Gene name |
|---|---|---|
| P00491 | Purine nucleoside phosphorylase | PNP |
| P18206 | Vinculin | VCL |
| P14384 | Carboxypeptidase M | CPM |
| Q96KX1 | Uncharacterized protein C4orf36 | C4orf36 |
| Q93050 | V-type proton ATPase 116 kDa subunit a isoform 1 | ATP6V0A1 |
| Q93052 | Lipoma-preferred partner | LPP |
| Q13576 | Ras GTPase-activating-like protein IQGAP2 | IQGAP2 |
| Q8IWA5 | Choline transporter-like protein 2 | SLC44A2 |
| Q15847 | Adipogenesis regulatory factor | ADIRF |
| Q9Y5Z4 | Heme-binding protein 2 | HEBP2 |
| Q96CW1 | AP-2 complex subunit mu | AP2M1 |
| Q9BX97 | Plasmalemma vesicle-associated protein | PLVAP |
| P26038 | Moesin | MSN |
| P11277 | Spectrin beta chain, erythrocytic | SPTB |
| Q92692 | Poliovirus receptor-related protein 2 | PVRL2 |
| Q15286 | Ras-related protein Rab-35 | RAB35 |
| Q01995 | Transgelin | TAGLN |
| P15586 | N-acetylglucosamine-6-sulfatase | GNS |
| Q9UL25 | Ras-related protein Rab-21 | RAB21 |
| P05106 | Integrin beta-3 | ITGB3 |
| P13987 | CD59 glycoprotein | CD59 |
| P06703 | Protein S100-A6 | S100A6 |
| O43488 | Aflatoxin B1 aldehyde reductase member 2 | AKR7A2 |
| Q13423 | NAD(P) transhydrogenase, mitochondrial | NNT |
| Q9UK41 | Vacuolar protein sorting-associated protein 28 homolog | VPS28 |
| Q06495 | Sodium-dependent phosphate transport protein 2A | SLC34A1 |
| Q9Y266 | Nuclear migration protein nudC | NUDC |
| Q7LBR1 | Charged multivesicular body protein 1b | CHMP1B |
| O43707 | Alph-actinin-4 | ACTN4 |
| Q9UBV8 | Peflin | PEF1 |

Among the 106 proteins found to be increased in the expression, the proteins shown in Table 3 including azurocidin (hereinafter referred to as AZU1) are characteristic with a large difference in the expression level from the proteins contained in the Te-EVs derived from the normal tissue, and hence can be more preferably used as the markers for renal cell carcinoma.

[Table 3]

| Protein Description | Gene name | AC number |
|---|---|---|
| Glycogenin-1 | GYG1 | P46976 |
| Azurocidin | AZU1 | P20160 |

(continued)

| Protein Description | Gene name | AC number |
|---|---|---|
| Carbonic anhydrase 9 | CA9 | Q16790 |
| Catechol O-methyltransferase | COMT | P21964 |
| Starch-binding domain-containing protein 1 | STBD1 | O95210 |
| Phosphoprotein associated with glycosphingolipid-enriched microdomains 1 | PAG1 | Q9NWQ8 |
| Tissue factor | F3 | P13726 |
| Thymidine phosphorylase | TYMP | P19971 |
| Leukocyte-associated immunoglobulin-like receptor 1 | LAIR1 | Q6GTX8 |
| Transmembrane glycoprotein NMB | GPNMB | Q14956 |
| Glutamine--fructose-6-phosphate aminotransferase [isomerizing] 1 | GFPT1 | Q06210 |
| Glycogen [starch] synthase, muscle | GYS1 | P13807 |
| High affinity immunoglobulin epsilon receptor subunit gamma | FCER1G | P30273 |
| Versican core protein | VCAN | P13611 |
| Granulins | GRN | P28799 |
| Prostaglandin F2 receptor negative regulator | PTGFRN | Q9P2B2 |
| Solute carrier family 2, facilitated glucose transporter member 3 | SLC2A3 | P11169 |
| Tyrosine-protein phosphatase non-receptor type substrate 1 | SIRPA | P78324 |
| Nucleophosmin | NPM1 | P06748 |
| Receptor-type tyrosine-protein phosphatase C | PTPRC | P08575 |
| Density-regulated protein | DENR | O43583 |
| DNA-(apurinic or apyrimidinic site) lyase | APEX1 | P27695 |
| Cytochrome P450 2C8 | CYP2C8 | P10632 |
| Vimentin | VIM | P08670 |
| CAP-Gly domain-containing linker protein 1 | CLIP1 | P30622 |
| Carbonic anhydrase 3 | CA3 | P07451 |

In particular, carbonic anhydrase 9, catechol O-methyltransferase, phosphoprotein associated with glycosphingolipid-enriched microdomains 1, leukocyte-associated immunoglobulin-like receptor 1, transmembrane glycoprotein NMB, high affinity immunoglobulin epsilon receptor subunit gamma, solute carrier family 2, facilitated glucose transporter member 3, tyrosine-protein phosphatase non-receptor type substrate 1, receptor-type tyrosine-protein phosphatase C, vimentin and carbonic anhydrase 3 are regarded as particularly useful markers because these have been reported to be highly expressed in renal cancer. Besides, phosphoprotein associated with glycosphingolipid-enriched microdomains 1 and tyrosine-protein phosphatase non-receptor type substrate 1 are regarded as promising candidates for a target for drug discovery because these make contribution to inhibition of T cell activity and immune evasion by cancer cells when highly expressed. One of these markers may be singly used, or when a plurality of markers are used in combination, the specificity and the sensitivity can be increased. Further, an existing biomarker may be used in combination for diagnosis.

Although the analysis results of the proteins contained in the Te-EVs are herein described, any biological component contained in the Te-EVs may be analyzed to be used as a biomarker. Examples of such a biological component include a nucleic acid and a lipid.

[Example 4] Analysis of AZU1

The results of detailed analysis of AZU1 farthest from the origin of the volcano plot ($p = 2.85 \times 10^{-3}$, fold-change: 31.59, shown with an arrow AZU1 in Figure 5) will now be described, and it goes without saying that the other proteins described above can be similarly used as the markers.

Figure 6A illustrates the concentrations of AZU1 in the Te-EVs derived from the normal tissue and the tumor tissue of 20 cases of renal cell carcinoma patients. In all the 20 renal cell carcinoma patients used for the analysis by the mass spectrometry, the expression of AZU1 increased in the Te-EVs derived from the tumor tissue. Figure 6B illustrates the concentrations of AZU1 depending on the progression of the cancer, and as renal cell carcinoma progressed, the amount of AZU1 contained in the Te-EVs increased. In a cancerous region of renal cell carcinoma at stage T3 (T3a and T3b), the content of AZU1 was significantly higher than in a non-cancerous region. Besides, as illustrated in an enlarged view in Figure 6B, the content of AZU1 was different even in early stages of cancer (T1a to T2a) as compared with that in the Te-EVs derived from the normal tissue.

Besides, the increase of the expression of AZU1 in accordance with the progression of cancer was checked by the Western blotting (Figure 6C). Five hundred ng per lane of EVs protein was separated by electrophoresis and then transferred onto a film, and the detection was performed in the same manner as in Example 2 by using an anti-AZUI monoclonal antibody (manufactured by Abcam Plc.) as a primary antibody. As a result of the analysis of Te-EVs obtained from four patients at stages of T1a to T3b, it was confirmed, in all samples, that the expression of AZU1 remarkably increased in Te-EVs obtained from a cancerous region (T) as compared with Te-EVs obtained from a non-cancerous region (N).

It has been reported that extracellular vesicles derived from cancer are detected also in a serum (Non Patent Literatures 3 to 5 and 11 to 13). Therefore, the amount of AZU1 in EVs contained in a serum sample was measured by quantitative mass spectrometry (Figure 6D).

The EVs contained in the serum sample was purified by using an EVSecond column (manufactured by GL Sciences Inc.). AZU1 was not at all detected in EVs contained in serums of 10 cases of healthy persons, but AZU1 was detected in 10 cases out of 19 cases of renal cell carcinoma patients. Although the AZU1 content did not increase in accordance with the progression of cancer as in the Te-EVs derived from the renal cell carcinoma tissue, it was detected at a high ratio in EVs contained in serums of cancer patients at early stages of T1a to T2b. This result reveals that a renal cell carcinoma test for detecting AZU1 using a serum is effective.

That AZU1 was detected in the extracellular vesicles present in the serum of a renal cell carcinoma patient indicates that a protein different in the content in Te-EVs can be detected in the serum. Accordingly, it indicates that a biomarker found by this method can function as a disease marker with which a test can be performed by using a serum.

[Example 5] Analysis of Effect of AZU1 in Renal Cell Carcinoma

Since the expression of AZU1 was detected specifically to the renal cell carcinoma tissue, the biological effect of AZU1 was examined. It is known that angiogenesis is generated largely to construct microenvironment of tumor tissue in renal cell carcinoma (Non Patent Literatures 14 to 17). Therefore, the effect of AZU1 on the form of vascular endothelial cells was examined.

First, localization of intrinsic AZU1 was examined under an immunoelectron microscope. The localization of AZU1 was examined in Te-EVs obtained from normal tissue and tumor tissue of the same patient by using an anti-AZU1 antibody obtained by immunizing a rabbit (manufactured by Abcam Plc.) and an anti-CD9 monoclonal antibody as primary antibodies, and secondary antibodies labeled with colloidal gold particles having different sizes (Figure 7A).

An antibody labeled with 40 nm colloidal gold particles was used as an anti-rabbit antibody, and an antibody labelled with 20 nm colloidal gold particles was used as an anti-mouse antibody. Accordingly, a large particle corresponds to the expression of AZU1 and a small particle corresponds to the expression of CD9 in Figure 7A. Although the expression of CD9 was found on the surface of the Te-EVs obtained from either of the normal tissue and the tumor tissue, the expression of AZU1 was found on the surface of the Te-EVs derived from the tumor tissue alone.

The AZU1 expression in a renal cell carcinoma cell line was checked by the Western blotting (Figure 7B). The expression of AZU1 in EVs isolated from 786-O, ACHN, Caki-1 and Caki-2 (obtained from ATCC) cell lines and in a cell lysate (whole cell lysate) was examined. The expression of AZU1 was found in any of these cells, and although AZU1 was detected in the EVs isolated from culture fluids (cultured media EVs, hereinafter sometimes referred to as CM-EVs) of the three cell lines of 786-O, Caki-1 and Caki-2, AZU1 was minimally detected in CM-EVs derived from the ACHN cell line.

Next, the permeability of the vascular endothelial cells of the CM-EVs obtained from these cell lines was analyzed based on transendothelial electrical resistance (TEER) (Figure 7C). The TEER was measured by using HUVEC cells (obtained from Gibco). After seeding $4.0 \times 10^4$ HUVEC cells in a 24-well culture insert (manufactured by Thermo Fisher Scientific K.K.) with a pore size of 0.4 $\mu$m and culturing the cells for 4 days, CM-EVs obtained from each cell line was added thereto in a concentration of 10 $\mu$g/ml, and the TEER was measured with Millicell(R) ERS-2 voltammeter (manufactured by Millipore) to calculate the TEER of each sample in accordance with the following expression:

[Expression 1]

$$(\text{Electric resistance } (\Omega) \text{ of sample well} - \text{Electric resistance } (\Omega) \text{ of empty well}) \times \text{culture area } (cm^2) = \text{TEER } (\Omega cm^2)$$

The results are illustrated in Figure 7C. As a result of measuring the TEER over time, the TEER of the HUVEC cell sheet was reduced 24 hours after the addition of the CM-EVs. In particular, the TEER was found to be remarkably reduced by addition of CM-EVs obtained from the Caki-1 and 786-O cells in which a larger amount of AZU1 was presented on EVs.

Since it was presumed that the EVs having a larger amount of AZU1 presented thereon had an effect of increasing permeability, detailed analysis was performed by using a system in which AZU1 was forcedly expressed. AZU1-FLAG (manufactured by Addgene) was introduced into and forcedly expressed in ACHN cells in which AZU1 was minimally detected in CM-EVs. As illustrated in Figure 7D, in the ACHN cell line in which AZU1-FLAG was forcedly expressed, it was found that EVs also contained a large amount of AZU1-FLAG.

Besides, it was confirmed under an immunoelectron microscope that the AZU1-FLAG was presented also on the EVs. The detection of the FLAG was performed by using an anti-FLAG monoclonal antibody (manufactured by Sigma-Aldrich) as a primary antibody and using a colloidal gold labeled anti-mouse antibody (Figure 7E). Although the FLAG was not detected in the CM-EVs obtained from cells to which a vector alone was introduced, the FLAG was detected on the CM-EVs obtained from cells into which the AZU1-FLAG was introduced. It was confirmed based on these results that the forcedly expressed AZU1-FLAG was also presented on the EVs in the same manner as the intrinsic AZU1.

The thus obtained expression system was used to examine the permeability by using HUVEC (Figure 7F). It was clarified that CM-EVs obtained from cells in which AZU1 is forcedly expressed remarkably reduces the TEER as compared with CM-EVs obtained from cells into which a vector alone is introduced. Accordingly, it is suggested that AZU1 has an effect of disintegrating the form of vascular endothelial cells.

Next, examination was made to check whether or not a similar effect can be exhibited by Te-EVs obtained from renal cell carcinoma patients. The permeability of cells was measured by using Te-EVs derived from normal tissue and tumor tissue obtained from six patients at various stages. Figure 8A illustrates change, over time, of the TEER in each patient after addition of Te-EVs derived from the normal tissue and the tumor tissue, and Figure 8B is a diagram in which the respective measurement values are plotted together. When the Te-EVs derived from the tumor tissue were added, the TEER was found to be remarkably reduced from 12 hours after the addition. Besides, when the Te-EVs obtained from a patient with cancer in an advanced stage were added, the TEER was more remarkably reduced.

Examinations were made to check whether or not the difference in the effect on the TEER of the Te-EVs derived from the normal tissue and the tumor tissue was caused by incorporation into cells of the Te-EVs derived from these tissues. The Te-EVs derived from the normal tissue and the tumor tissue were respectively labeled with PKH-67 and PKH-26 (manufactured by Sigma Aldrich) and then mixed, the resultant mixture was added to a culture fluid of HUVEC cells, and the resultant was observed under a microscope 12 hours later. Figure 8C illustrates the results obtained by using the Te-EVs obtained from two patients. In either sample, Te-EVs derived from the normal tissue looking bright around a nucleus (stained green under a fluorescence microscope) and Te-EVs derived from the tumor tissue recognized as a dark stained image (stained red under a fluorescence microscope; two portions are shown with arrows in each image) were both detected to the same extent. Accordingly, it was confirmed that the incorporation efficiency of Te-EVs did not vary depending on the tissue from which the Te-EVs were derived. In other words, it was revealed that the reduction of the TEER caused by the Te-EVs derived from the tumor tissue was not caused by a difference in the incorporation efficiency of Te-EVs. Therefore, the reduction is probably caused by proteins and the like including AZU1 presented on the Te-EVs.

In blood of a patient, Te-EVs derived from tumor tissue and normal tissue are circulating in a mixed state. It was examined whether or not the reduction of the TEER was induced in such a mixed state. Figure 8D illustrates the TEER measured over time after adding, to a culture fluid of HUVEC cells, a mixture of Te-EVs derived from normal tissue and tumor tissue obtained from three patients. The reduction of the TEER was observed in the Te-EVs obtained from any one of the patients even if the mixture of the Te-EVs derived from tumor tissue and normal tissue was used. This result seems to reflect a phenomenon actually occurring in a body of a patient, and hematogenous metastasis seems to be induced by AZU1.

Based on the above-described results, it is presumed that AZU1 is a disease-related gene closely involved in the onset and development of renal cell carcinoma. Accordingly, a therapeutic agent can be created by using AZU1 as a target. In this manner, when the function of a biological component such as a protein or a nucleic acid expressed specifically to a disease is analyzed, a target of a novel therapeutic agent can be found.

[Example 6] Analysis using Serum of Patient

If a protein characteristic to a renal cell carcinoma patient can be detected with EVs contained in a serum, it can be used as a useful marker for early detection of renal cell carcinoma. Therefore, proteins different between a renal cell carcinoma patient and a healthy person were searched for in EVs contained in a serum. Table 4 shows proteins that can be detected in EVs contained in a serum of a renal cell carcinoma patient but cannot be detected in those of a heathy person, excluding those detected as a result of the Te-EVs analysis shown in Tables 1 and 2.

[Table 4]

| AC number | Protein Description | Gene name |
|---|---|---|
| P11678 | Eosinophil peroxidase | EPX |
| Q8NI35 | InaD-like protein | INADL |
| P68104 | Elongation factor 1-alpha 1 | EEF1A1 |
| Q63ZY3 | KN motif and ankyrin repeat domain-containing protein 2 | KANK2 |
| Q16836 | Hydroxyacyl-coenzyme A dehydrogenase, mitochondrial | HADH |
| P07437 | Tubulin beta chain | TUBB |
| P55058 | Phospholipid transfer protein | PLTP |
| Q9Y3R5 | Protein dopey-2 | DOPEY2 |
| P05543 | Thyroxine-binding globulin | SERPINA7 |
| P06732 | Creatine kinase M-type | CKM |
| P31946 | 14-3-3 protein beta/alpha | YWHAB |
| O75533 | Splicing factor 3B subunit 1 | SF3B1 |
| P07900 | Heat shock protein HSP 90-alpha | HSP90AA1 |
| Q15404 | Ras suppressor protein 1 | RSU1 |
| Q9UII5 | Zinc finger protein 107 | ZNF107 |
| P09874 | Poly [ADP-ribose] polymerase 1 | PARP1 |
| P10619 | Lysosomal protective protein | CTSA |
| P14618 | Pyruvate kinase PKM | PKM |
| P14625 | Endoplasmin | HSP90B1 |
| P23284 | Peptidyl-prolyl cis-trans isomerase B | PPIB |
| P60033 | CD81 antigen | CD81 |
| P62249 | 40S ribosomal protein S16 | RPS16 |
| Q86XI8 | Uncharacterized protein C19orf68 | C19orf68 |

These proteins are characteristic to a renal cell carcinoma patient, but are not detected in all renal cell carcinoma patients. When a plurality of these markers are combined, however, a renal cell carcinoma patient can be detected at an early stage by using a blood sample. In addition to findings obtained from Te-EVs of renal cell carcinoma patients, these proteins specifically detected in the serums of the renal cell carcinoma patients are usable as novel markers for renal cell carcinoma, for which an effective biomarker has not been found.

Industrial Applicability

According to a search method for a biomarker of the present invention, a tissue-specific disease marker contained in extracellular vesicles can be obtained. Since extracellular vesicles are secreted into a body fluid, they are very useful as non-invasive or minimally invasive disease markers. When this method is employed, a biomarker can be obtained for a disease for which an effective biomarker has not been found.

Besides, a renal cell carcinoma marker described herein can be used, in a renal cell carcinoma detecting test, as a novel marker for renal cell carcinoma for which there has been no effective biomarker. Furthermore, since AZU1 has an effect of disintegrating the form of vascular endothelial cells, it is suggested to be significant for metastasis of cancer cells. Therefore, a molecular target drug using AZU1 as a target is expected to have an anticancer metastasis effect.

**Claims**

1.  A search method, comprising:

    immersing resected diseased tissue in an immersion liquid;
    analyzing an exuded component derived from the diseased tissue exuded from the diseased tissue in the immersion liquid; and
    identifying a biomarker and/or disease-related gene derived from the diseased tissue.

2.  The search method according to claim 1, comprising:

    immersing normal tissue obtained from around the resected diseased tissue in an immersion liquid;
    analyzing an exuded component derived from the normal tissue exuded from the normal tissue in the immersion liquid;
    identifying a biomarker and/or disease-related gene derived from the normal tissue; and
    selecting a disease-specific biomarker and/or disease-related gene by comparatively examining the biomarker and/or disease-related gene derived from the diseased tissue and the biomarker and/or disease-related gene derived from the normal tissue.

3.  The search method according to claim 1 or 2,
    wherein the exuded component is an extracellular vesicle, a protein, a nucleic acid or a lipid.

4.  The search method according to any one of claims 1 to 3,
    wherein the exuded component is an extracellular vesicle, and
    the biomarker and/or disease-related gene contained in the extracellular vesicle is a protein, a nucleic acid or a lipid.

5.  The search method according to any one of claims 1 to 4,
    wherein the disease is cancer, neurodegenerative disease, multiple sclerosis, diabetes, liver disease, autism or cerebral infarction.

6.  A test method for renal cell carcinoma, comprising:

    separating an extracellular vesicle contained in a body fluid;
    detecting at least one biomarker out of biomarkers listed in Tables 1, 2 and 4 contained in the extracellular vesicle; and
    comparing with a prescribed value.

7.  The test method for renal cell carcinoma according to claim 6,
    wherein the biomarker is AZU1, CA9, STBD1, COMT or GYG1.

8.  The test method for renal cell carcinoma according to claim 6 or 7,
    wherein the body fluid is blood or urine.

9.  Biomarkers for renal cell carcinoma, listed in Tables 1, 2 and 4.

10. A method for screening a therapeutic agent for renal cell carcinoma using AZU1, CA9, STBD1, COMT or GYG1 as a target, comprising:
    selecting a candidate compound by using AZU1, CA9, STBD1, COMT or GYG1 as an index.

# Figure 1

**Cancerous region**

**Normal region**

Serum-free medium

Incubation

**Tissue-exudative extracellular vesicles (Te-EVs)**

2,000 × g, 30 min

16,000 × g, 30 min

100,000 × g, 90 min (×2)

**Tryprin digestion**

**LC/MS analysis**

*HPLC*  *Mass Spec.*

**Protein identification &quantification (MaxQuant)**

**Statistical analysis**

## Figure 2

# Figure 3

Figure 4A

Total protein IDs
3871
Normal — Tumor
160   3458   253

Figure 4B

**CC : Total proteins**
Plasma membrane
Cytosol
Cytoskeleton
Organelle membrane
Endoplasmic reticulum
Mitochondrion
(%) 0 5 10 15 20

**CC : Normal specific**
Organelle envelope
Extracellular matrix
Mitochondrial membrane
NADH dehydro complex
Mitochondrial respiratory
Proton-transport complex
(%) 0 5 10 15 20 25

**CC : Tumor specific**
Integral membrane
Endoplasmic reticulum
Insoluble fraction
Lysosome
Vacuole
Spindle pole
(%) 0 10 20 30 40 50 60

Figure 4C

**BP : Total proteins**
Protein localization
Protein transport
Proteolysis
Vesicle transport
Response to stimulus
Oxidation reduction
(%) 0 2 4 6 8 10

**BP : Normal specific**
Transmembrane transport
Oxidation reduction
Protein localization
Generation of metabolites
Oxidative phosphorylation
Response to stimulus
(%) 0 5 10 15 20

**BP : Tumor specific**
Protein localization
Homeostasis
Protein catabolism
Fatty acid metabolism
Monosaccharide metabolism
Induction of apoptosis
(%) 0 5 10 15 20

Figure 4D

**MF : Total proteins**
Nucleotide binding
Calcium ion binding
Structural molecule activity
Cytoskeleton binding
Enzyme binding
Peptidase activity
(%) 0 5 10 15 20 25

**MF : Normal specific**
Cofactor binding
Symporter activity
NADH dehydro activity
Sodium ion binding
Anion transporter
NES receptor activity
(%) 0 5 10 15 20 25

**MF : Tumor specific**
Nucleotide binding
Actin binding
Enzyme inhibitor
Carboxylic acid binding
Anion binding
Hydrolase activity
(%) 0 10 20 30 40 50

## Figure 5

## Figure 6

### Figure 6A

AZU1 level in EVs

### Figure 6B

AZU1 level in EVs

### Figure 6C

### Figure 6D

AZU1 in Serum EVs

# Figure 7

## Figure 7A

**Normal Te-EV**
**(Sample No. 5)**

**Tumour Te-EV**
**(Sample No. 5)**

## Figure 7B

## Figure 7C

## Figure 7D

## Figure 7E

mock EV          AZU1-FLAG EV

## Figure 7F

# Figure 8

**Figure 8A**

**Figure 8B**

**Figure 8C**

Mixed Te-EV (Sample No. 13)    Mixed Te-EV (Sample No. 20)

**Figure 8D**

## INTERNATIONAL SEARCH REPORT

| International application No. |
|---|
| PCT/JP2017/038812 |

**A. CLASSIFICATION OF SUBJECT MATTER**
Int.Cl. G01N33/68(2006.01)i, C12Q1/68(2018.01)i, G01N33/15(2006.01)i, G01N33/50(2006.01)i, G01N33/574(2006.01)i, G01N33/92(2006.01)i

According to International Patent Classification (IPC) or to both national classification and IPC

**B. FIELDS SEARCHED**

Minimum documentation searched (classification system followed by classification symbols)
Int.Cl. G01N33/68, C12Q1/68, G01N33/15, G01N33/50, G01N33/574, G01N33/92

Documentation searched other than minimum documentation to the extent that such documents are included in the fields searched
Published examined utility model applications of Japan 1922–1996
Published unexamined utility model applications of Japan 1971–2017
Registered utility model specifications of Japan 1996–2017
Published registered utility model specifications of Japan 1994–2017

Electronic data base consulted during the international search (name of data base and, where practicable, search terms used)
JSTPlus/JMEDPlus/JST7580 (JDreamIII), CAplus/MEDLINE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | 高橋枝里，血液細胞外小胞を対象とした腎細胞がんと膀胱がんのバイオマーカータンパク質の探索，日本プロテオーム学会大会プログラム・抄録集, 28 July 2016, page. 164, non-official translation (TAKAHASHI, Eri, "Search for biomarker proteins of renal cell carcinoma and bladder cancer for blood extracellular vesicles", Japan Proteome Society Convention Program and Abstracts) | 1-5 |
| A | 神宮司健太郎，淡明細胞型腎細胞癌組織由来 extracellular vesicles の解析，日本癌学会学術総会抄録集, 2015, vol.74th, page. P-3192, (JINGUJI, Kentaro, "Analysis of extracellular vesicles derived from clear cell type renal cell carcinoma tissue", Abstracts of Academic Societies of the Japanese Cancer Association) | 1-5 |

☒ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 22 January 2018 (22.01.2018) | 30 January 2018 (30.01.2018) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2017/038812 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2012-508577 A (CARIS LIFE SCIENCES LUXEMBOURG HOLDINGS) 12 April 2012, paragraphs [0224], [0225], [0646] & WO 2010/056337 A2 paragraphs [00223], [00572], [00573] & US 2010/0113290 A1 & EP 2350320 A2 & CN 102301002 A | 1-5 |
| X<br>Y | 果然，配線癌細胞の分泌蛋白質群一外科的切除組織の培養上清に含まれる診断バイオマーカー候補の同定一，肺癌，2010, vol. 50, no. 2, pp.141-150, 141, non-official translation (KA, Zen, "Secretory protein group of pulmonary adenocarcinoma cell - Identification of biomarkers included in culture supernatant of surgically resected tissue", Lung Cancer) | 1-3, 5<br>4 |
| X<br>Y | 果然，原発性肺腺がん腫瘍組織の培養上清のプロテオーム解析，東京医科大学雑誌，30 October 2009, vol. 67, no. 4, pp. 502 P3-41, (The Journal of Tokyo Medical University), non-official translation (KA, Zen, "Proteome analysis for culture supernatant of primary pulmonary adenocarcinoma tumor tissue") | 1-3, 5<br>4 |
| Y | 森田卓也，Liquid biopsyに応用可能なEwing肉腫細胞由来のexosome表面マーカーの同定，日本整形外科学会雑誌，10 September 2015, vol. 89 No. 8, pp. S1796 2-7-22, (The Journal of Japanese Orthopaedic Surgical Society), non-official translation (MORITA, Takuya, "Identification of exosome surface marker derived from Ewing sarcoma cell applicable to liquid biopsy") | 4 |
| A | 川上降雄，バイオマーカーのプロテオミクス：液体クロマトグラフィー／タンデム質量分析を用いたプロテオーム統計解析法の開発とその応用，JSBSM Lett, vol. 35, no. 1, 25 March 2010, pp. 4-12, abstract, (KAWAKAMI, Furuo, "Proteomics of biomarker: Proteome statistic analysis using liquid chromatography/tandem mass spectrometry, and applications thereof") | 1-5 |
| A | ZWICKL, Hannes, A novel technique to specifically analyze the secretome of cells and tissues, Electrophoresis, July 2005, vol. 26, no. 14, pp. 2779-2785, abstracts, 1., 2. 1, 3. | 1-5 |
| A | 清海杏奈，3次元ヒト乳癌組織から分泌される免疫作用物質及びその抑制性Ｔ細胞動態への影響，日本薬学会年会要旨集，2014, vol. 134th, page. ROMBUNNO. 30PMM-089, non-official translation (KIYOMI, Anna, "Immunologic substance secreted from 3-dimensional human breast cancer, and effects on behavior of suppressor T cell thereof", Abstracts of The Pharmaceutical Society of Japan Annual Meeting) | 1-5 |

Form PCT/ISA/210 (continuation of second sheet) (January 2015)

## INTERNATIONAL SEARCH REPORT

International application No.

PCT/JP2017/038812

C (Continuation).  DOCUMENTS CONSIDERED TO BE RELEVANT

| Category* | Citation of document, with indication, where appropriate, of the relevant passages | Relevant to claim No. |
|---|---|---|
| A | BONIFATI, C., IL-1alpha, IL-1beta and psoriasis: conflicting results in the literature. Opposite behavior of the two cytokines in lessional or non-lessional extracts of whole skin, J Biol Regul Homeost Agents, 1997, vol. 11, no. 4, pp. 133-136, MATERIALS AND METHODS | 1-5 |
| A | KIYOHARA, Yumiko, Drug screening and grouping by sensitivity with a panel of primary cultured cancer spheroids derived from endometrial cancer, Cancer Science, April 2016, vol. 107, no. 4, pp.452-460, abstract | 1-5 |
| A | BERGMAN A-C, Identification of gel-separated tumor marker proteins by mass spectrometry, Electrophoresis, February 2000, vol. 21, no. 3, pp. 679-686, Materials and Methods | 1-5 |
| A | JP 2013-544361 A (KCI LICENSING, INC.) 12 December 2013, paragraph [0029] & WO 2012/071516 A2 paragraph [0031] & US 2012/0129186 A1 & US 2015/0346216 A1 & EP 2643693 A2 & CN 103097892 A | 1-5 |
| A | EP 2955232 A1 (BORK, Peer) 16 December 2015, examples (Family: none) | 1-5 |
| P, X | 長山聡，切除組織培養分泌エクソソームの網羅的解析による大腸癌診断バイオマーカーの探索，日本外科学会定期学術集会， 2017, vol. 117th, page. ROMBUNNO. PS-110-7, session 27-29 April 2017, (Annual Congress of Japan Surgical Society), non-official translation (NAGAYAMA, Satoshi, "Search for biomarkers for diagnosis of colorectal cancer by comprehensive analysis of resected tissue culture secretion exosome") | 1-5 |
| P, X | 村岡賢,切除組織培養分泌細胞外小胞の網羅的解析による大腸癌早期診断バイオマーカーの開発，日本プロテオーム学会大会プログラム·抄録集， 2017, vol. 2017, page. 148, session 26-28 July 2017, (Proteome-wide profiling of viable colorectal cancer tissue-derived extracellular vesicle), non-official translation (MURAOKA, Ken, Japanese Protemics Society: abstracts) | 1-5 |

Form PCT/ISA/210 (continuation of second sheet) (January 2015)

## INTERNATIONAL SEARCH REPORT

| International application No. |
|---|
| PCT/JP2017/038812 |

**Box No. II      Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.: 1-5

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

| International application No. |
| --- |
| PCT/JP2017/038812 |

"Continuation of Box No. III"

The special technical feature of the invention as in claim 1 is a search method characterized in that a resected diseased tissue is immersed in an immersion liquid, and diseased tissue-derived exudate components exuded from the diseased tissue in the immersion liquid are analyzed to identify a biomarker derived from the diseased tissue and/or a disease-related gene, whereas the provisional special technical feature of the invention as in claims 6-9 involves separating extracellular vesicles contained in body fluids, and using at least one of the biomarkers listed in Tables 1, 2 and 4 and contained in the vesicles as a biomarker for testing renal cell carcinoma, and the special technical feature of the invention as in claim 10 is a method for screening a therapeutic agent which is for renal cell carcinoma and targets AZU1, CA9, STBD1, COMT, and GYG1, wherein the method is characterized in that AZU1, CA9, STBD1, COMT, and GYG1 are used as indicators to select candidate compounds. The special technical features of the invention as in claims 6-9 and claim 10 are not the same as the special technical feature of the invention as in claim 1.

In addition, with regard to the invention as in claims 6-9, documents 1-3 indicate that renal cell carcinoma is detected by a biomarker present in extracellular vesicles in body fluids. Thus, in the invention as in claims 6-9, the feature, in which extracellular vesicles contained in body fluids are separated, and at least one of the biomarkers which are listed in Tables 1, 2 and 4 and contained in the vesicles is used as a biomarker for testing renal cell carcinoma, is not recognized as a special technical feature of the invention.

Thus, claims 6-9 include 420 inventions having the respective biomarkers listed in Tables 1, 2, and 4 as special technical features, and the inventions having the special technical features of these respective biomarkers do not share a common special technical feature.

**INTERNATIONAL SEARCH REPORT**

International application No.

PCT/JP2017/038812

Therefore, claims 1-10 of the present application include the inventions below.

(Invention 1) The invention as in claims 1-5, characterized in that a resected diseased tissue is immersed in an immersion liquid, and diseased tissue-derived exudate components exuded from the diseased tissue in the immersion liquid are analyzed to identify a biomarker derived from the diseased tissue and/or a disease-related gene.

(Inventions 2-241) The inventions selectively set forth in claims 6-9, wherein the respective biomarkers listed in Tables 1, 2 and 4 are used as biomarkers for testing renal cell carcinoma.

(Invention 422) The invention as in claim 10, which is a method for screening a therapeutic agent which is for renal cell carcinoma and targets AZU1, CA9, STBD1, COMT, and GYG1, wherein the method is characterized in that AZU1, CA9, STBD1, COMT, and GYG1 are used as indicators to select candidate compounds.

Document 1: 高橋枝里, 血液細胞外小胞を対象とした腎細胞がんと膀胱がんのバイオマーカータンパク質の探索, 日本プロテオーム学会大会プログラム·抄録集, 28 July 2016, Page 164, non-official translation (TAKAHASHI, Eri. Search for biomarker proteins of renal cell carcinoma and bladder cancer for blood extracellular vesicles, Japan Proteome Society Convention Program and Abstracts)

Document 2: 神宮司健太郎, 淡明細胞型腎細胞癌組織由来 extracellular vesicles の解析, 日本癌学会学術総会抄録集, 2015, Vol. 74th, Page P-3192, non-official translation (JINGUSHI, Kentaro. Analysis of extracellular vesicles derived from clear cell type renal cell carcinoma tissue, Abstracts of Academic Societies of the Japanese Cancer Association)

Document 3: JP 2012-508577 A (Caris Life Sciences Luxembourg Holdings) 12 April 2012, paragraphs [0224] [0225] [0646] & WO 2010/056337 A2 (paragraphs [00223], [00572], [00573]) & US 2010/0113290 A1 & EP 2350320 A2 & CN 102301002 A

Form PCT/ISA/210 (extra sheet) (January 2015)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- JP 2016086678 A
- JP 2013140030 A
- JP 2015505965 A

## Non-patent literature cited in the description

- **KAHLERT, C. ; KALLURI, R.** *J. Mol. Med.,* 2013, vol. 91 (4), 431-437
- **AN, T. et al.** *J. Extracell. Vesicles.,* 2015, vol. 4, 27522
- **PEINADO, H. et al.** *Nat. Med.,* 2012, vol. 18 (6), 883-891
- **COSTA-SLIVA, B. et al.** *Nat. Cell Biol.,* 2015, vol. 17 (6), 816-826
- **HOSHINO, A. et al.** *Nature,* 2015, vol. 527 (7578), 329-335
- **LASSER, C. et al.** *J. Vis. Exp.,* 2012, vol. 59, e3037
- **GEISSLER, K. et al.** *Oncoimmunology,* 2015, vol. 4, e985082
- **TELTSH, O. et al.** *Oncotarget,* 2015, vol. 32, 33191-33205
- **GULDUR, M. E. et al.** *J. Pak. Med. Assoc.,* 2014, vol. 3, 300-303
- **BUSSOLATI, B. et al.** *FASEB J.,* 2003, vol. 17 (9), 1159-1161
- **WIKLANDER, O.P. et al.** *J. Extracell. Vesicles,* 2015, vol. 4, 26316
- **TOMINAGA, N. et al.** *Adv. Drug Deliv. Rev.,* 2015, vol. 95, 50-55
- **LAI, C. P. et al.** *ACS Nano.,* 2014, vol. 8 (1), 483-494
- **GRUENWALD, V. et al.** *BMC Cancer,* 2010, vol. 10, 695
- **VROLING, L. et al.** *Angiogenesis,* 2009, vol. 12 (1), 69-79
- **DEL PUERTO-NEVADO, L. et al.** *Br. J. Cancer,* 2014, vol. 110, 2700-2707
- **SHANKHAJIT, D. et al.** *International Journal of Nutrition, Pharmacology, Neurological diseases,* 2012, vol. 2, 3-7